# EUROPEAN PATENT APPLICATION

(11) **EP 1 930 344 A1**
(43) Date of publication of application: **11.06.2008**
(21) Application number: 06810605.3
(22) Date of filing: 27.09.2006
(51) Int. Cl.: C07K 14/47, A61K 38/00, A61P 9/00, A61P 15/00, A61P 25/00, A61P 27/02, A61P 29/00, A61P 35/00, C12Q 1/02, G01N 33/15, G01N 33/50, C12N 15/09

(54) **POLYPEPTIDE HAVING ANTI-ANGIOGENIC ACTIVITY**

(30) Priority: 29.09.2005 JP 2005284270
(71) Applicant: SHIONOGI & CO., LTD., Osaka-shi, Osaka 541-0045 (JP); TOHOKU UNIVERSITY, Aoba-ku Sendai-shi, Miyagi 980-8577 (JP)
(72) Inventor: SATO, Yasufumi, c/o Tohoku University, Sendai-shi, Miyagi 9808577 (JP); SONODA, Hikaru, c/o Shionogi & Co.,Ltd., Osaka-shi, Osakai 541-0045 (JP); OHTA, Hideki, c/o Shionogi & Co.,Ltd., Osaka-shi, Osaka 541-0045 (JP)
(74) Representative: Vossius & Partner
(86) International application number: PCT/JP2006/319114
(87) International publication number: WO 2007/037245

(57) **Abstract**

The present inventors found out four kinds of fragments (42 kD, 36 kD, 32 kD, 27 kD) by forcibly expressing vasohibin in a vascular endothelial cell. The present inventors analyzed those fragments and, as a result, found out low molecular weight vasohibin which has an anti-angiogenic activity. The low molecular weight vasohibin is useful as a therapeutic agent for various diseases such as a vascular disease associated with angiogenesis, an inflammatory disease, an entoptic neovascular disease, a reproductive disease, a central nervous system disease and cancer and the like. In addition, the low molecular weight vasohibin is also useful as a marker for angiogenesis.

SEQUENCE LISTING

<110>  Tohoku University
       Shionogi & Co., LTD.

<120>  Polypeptide having anti-angiogenic activity

<130>  P1432 EP S3

<140>  EP 06 81 0605.3
<141>  2006-09-27

<150>  JP2005-284270
<151>  2005-09-29

<160>  2

<170>  PatentIn version 3.1

<210>  1
<211>  5747
<212>  DNA
<213>  Homo sapiens

<300>
<308>  NCBI/NM_014909
<309>  2004-08-23

<400>  1
ggctcccggc cagctgcgag tcttggctcc cggacttgtc tcgtcgcgtc ggagaaatcg      60

ccccccagcg ccgctctccc gcccgggggt cttggttccg agctcgcgcg gccgggagtc     120

gcctcggtct tccttggggc gcgcgcagat gtgagcgtgc gagagttgtg taggggattt     180

tgttccctcc gaaactgaga cccagggcgc ccagtgggca cccgtgcctt gactctgtcc     240

tttctgcagc cgctggtccg agctgtctgg cctcagtttc cctccgactt ttctccgctc     300

tgccagccct cactgctgcc cgtcattgtt ctcgcagtta gatgggggtg ctttgtgacg     360

gctgccaagt tggggtgtgt tctctttatt ccgtttttca aacagaacaa ggcctccaag     420

gctgacccca gacaacccac cccctcggac cctaattcac cttattgcac tgattttttt     480

tatcaagtcg tattttattg tacaggagcc acgccctgat ttcttaaagg cgccttgcac     540

tctggccatg tgttatctct gcagccggtg tgcgggaggc ctcttgtgag ccagttgttt     600

tcccgcctcc accacccccc tcgaagattt agggatgcca ggggggaaga aggtggctgg      660

gggtggcagc agcggtgcca ctccaacgtc cgctgcggcc accgccccct ctggggtcag    720

gcgtttggag accagcgaag gaacctcagc ccagagagat gaggagccag aagaggaagg     780

ggaagaggac ctgcgagacg gaggcgtccc cttctttgtc aaccggggtg ggctacctgt     840

ggatgaggcc acctgggaaa ggatgtggaa acacgtggcc aagatccacc ccgatggaga     900

gaaggtggcg caacggatcc gtggggccac agacctgccc aagatcccca taccgagtgt     960

gcctacgttc cagccgtcta cacctgtccc tgagcgcctg gaagctgtgc agcgctatat    1020

cagagagctg cagtacaatc acacagggac acagttcttt gaaattaaga agagcagacc    1080

tctgacaggg ctgatggacc tggccaagga aatgaccaaa gaggccctgc caatcaaatg    1140

cctggaagcc gtgatcctgg gaatttacct caccaacagc atgcccaccc tggagcgctt 1200
ccccatcagc ttcaagacct acttctcagg gaactacttc cgccacatcg tgctgggggt 1260
gaacttcgcg ggccgctacg gtgcgctggg catgagtcgg cgcgaggacc tgatgtacaa 1320
gccgcccgcc ttccgcacgc tcagcgagct cgtgctggac ttcgaggccg cctacggccg 1380
ctgctggcac gtgctcaaga aggtgaagct gggccagagc gtgtcacacg acccgcacag 1440
cgtggagcag atcgagtgga agcactcggt gctggacgtg gagcgcctgg gccgcgatga 1500
cttccgcaag gagctggagc gccacgcccg cgacatgcgg ctcaagattg gcaaagggac 1560
gggccctccc tctcccacca aggaccggaa gaaggatgtt tcttccccgc agcgggccca 1620
gtccagcccc caccgcagga acagccgcag tgaaagacgg ccctcgggtg acaagaagac 1680
ttccgagccc aaagccatgc cagaccttaa cgggtaccag atccgggtct gaggcggatg 1740
ccagcacccc aggccccacc cactcttggg ggccaggatc cacctgctgg aaccagcctt 1800
atgcatgggg aaggcggggc tggtgacaag gcagggcaag aggctgcagg aagagtgtgt 1860
tccagctcag ccccccaagc tgctctcgct cccactgagc caagccccct aactttgggc 1920
ctagaggccg ttagtatttt atttggagtt tttaactcta caactgaagt ttaaggtatt 1980
tggggaaaac ttagtccaaa tggatctgct gatggtggga aggccagtgc ttaacaaatc 2040
catgtgtcat ggggccaggt gagggaaact gctggttctg ctggtgcctc tgcccctggc 2100
ttccctctgg gagttgggtg catcttatca gtgggaaatc tcccagcctt accaggcctg 2160
tgatgggggg tgggggtggg gtggagatgt ttctccagtt ctgcctgccc tggcagaatc 2220
ttgacccagg gaaagggaag cagggtagga gtccttctga gaaaggtctg tgtagcccat 2280
taaccaggag cttggcacag gccacatctg ccccaagagc atgagctcgt ggctcaggag 2340
agccttcagg cccttgaggc ccccatgggc agtgctgtgt gggcagagga ggggtgatat 2400
gagagcgagc ccagggaagg acctctgggc aaaaagttcc caggccctaa ctgcgtctac 2460
ttgctcagtc ccagctctgc ctgttgctct agcccacagg ctcctgcgga gggtctgggc 2520
ttggcggagg acccagaatg gcactgaggc cagcatggct gtgggagttg agcaaaccct 2580
gggtgccagt ccaggagctg tggctggaca tggggtgatg gggcgggatg cttgggcctg 2640
ggtctctccg ccagcagtgc caggagccct tgctggggaa atcaagacca gactaggatg 2700
cttctgcccc aggcctgcct tccattcttt aacagcccat cttggcttgg ggttgcaatg 2760
atggctgggc cagtcacttg tggcagggca tcagggccct ggcagggaag aacctaggca 2820
cctggggttg tccccagcct gcccgtcagc atgagatacc cagtgggaaa gtgagaggat 2880
gcggagaggt tggcagagcc aggggtaggt tctggaggct caagcaacaa ggaggtgcag 2940
gtaaagggtg cagtgcagcc actgagggac agctgggaac tgggggatgc aagtgagaaa 3000
gggatgtggg ggagagttca ggatcaggct gcttgaggag taatgggtta cctacagcag 3060
agacgagatg gctgtttgtc aggaggcggt agaaaggtag aaaggattca gattgtggaa 3120
gggtaaatgg atgagatgac cattaaggtt ttgttttact gagaggctgt aagtctgcca 3180

gggacagctg tcagtaaggc tgcagaaggg ctggggggct acacaaggaa gagcagaact 3240
agggttgtaa gctcaaatga cgagcaagcg gtgagaagga ggcctgaagg gctgtgtggg 3300
gactatggct acctgaagag ggcacaccct gttaaagggg ccacagctgc tcagctgttc 3360
ttacagtgcc ggccctgtgt tgccagattg tctgctttgt cagaggccag aattctgact 3420
ttttatgtga aataggattt ttaaatgctg gtgatcactt aaaaaaaact taaaaaccca 3480
atactggcaa aagaggatgc cagtctgcaa tccctgaagt agagagagct cgtgctgggg 3540
agaagtccac caagatgctt tgaggcgggc tgtagcaaag tcctgtttct cagagctggg 3600
ctgggctggg gtaggatcct tgcagctgag gaggaggaaa agccactgag tctcctccca 3660
gagcgggaca aaccagaggc cctttgcagt tgctgggtca ccagcggggg tggcgcatgg 3720
agtacagaca gtgtagctct tggcctgcca gggagacgga tggtgccttt gaagcaaaga 3780
ggaagggaag gcagaaccag ggatgccttt gctgatgaga gtgcctgtca gggaaggcgc 3840
cacaggctgg cagctcttca aaccagcagc gcttgaccca gagccagatc cagcatggcc 3900
tggccagagg caccctggga ggccagctgg tcagtccctt gcctccccaa gttccccctg 3960
gggtcaatga gccctgggag gatgcctaac ctaactccag ccagactaat aggggcatgg 4020
tgacccttga ctcaccatcc catcccagct ttcagggagt gggggtagtg tggtctccat 4080
gttcctacta tgcctaagaa gagatggctc accttgggag gtgccaggct gaaactaggt 4140
cctttccggg tctggatgct gccgctcagg agcaggggcg tggcctcagc tgcctctggg 4200
agcttcccgg gaatgacagg gtttgagggg agtagatatg agagggagcc gctcctggtt 4260
ctggagtctt aggaggttcc aacttgcagg atcctttccc agagccctcc atggagaaaa 4320
cagcaaaatg aagcccttac ctgcttgctg tctgcaaggg agggagccga gccccagctg 4380
ataatccccc agcactcacc cttcctgagc tgaggcttcg gggctgtgga gaccagcaca 4440
ggacatagtg gtgcttttta aatttatttt taactgtttc tcatatgtag caacccctcc 4500
tcccctcctg ggcatgttta cacaggctct gctctggggg ctggcctggc tgtgaggttt 4560
ctggggaggc agagaggcag ggactttggg gccttagtca ccatccatgg tatcacctca 4620
tctcacttcc tgtgagggac agggcctggc tgatgtgatc ccagctcccc ccagttcagg 4680
actgtctttc agctcctttg cccctggagg tgggggctgc tggctgagga ggggtcaagg 4740
tgagttcaag aaagctacct gtggaaaatg gaccaggttg ggggggtgat tgcaaagtct 4800
ccccaaagcc tggctcctca tgctcagtgc caggggcaga acactgggga gccaggtata 4860
gagagccttc ctgtcataac tgccagtcct cttcctccaa ggcctctgca tattctcatg 4920
ttcccctcac ccatcatgcc agccacccct atccctcttc tagcagggcc aagatgggga 4980
cagtagcagc cctctggact tgggatgtga tgataaagca agcctagggc cagggtttgg 5040
ggagcagaga gagccaagaa gttgaccacg tgtgatttcc agcccttccc actgggactt 5100
gacttcccag gtcaaggagt ccgtctcatt ctggctggtc gagtgaccag aggcctgtgt 5160
gaatgtgtgc acctgctttt cctgcctgga atgttttctg gctcagctgc agcaacatct 5220

gtgagcccag tgtctgccct gtgtccctgg gctcgctcca agtgcaggaa cgtacatgca 5280

gggcccaaca tgatgatggt gtgaagggca ggaaacagtc ctctgaagga gtggggaggt 5340

gggcagtctg cccccgccag gtaccatcgc ctcctgccag cttccttaga ccaggcaggg 5400

ctgccatggt gctagctgca agtccatcag tattgaccgt ctcgctccat cttggccctc 5460

cggagtccca agtttccttt tcatcaaatc tgacaagaga gaagaaacat gggtgtgctt 5520

ggcccacagg gcctggtggt gatggacctc cccgctccct caagctctgg atggctgcag 5580

tgttgtacta gactttgttc aggctgttct catctcagta ttgccccttc ctttcacttt 5640

cacacttcat ctcattcctg ttgtcacttt ccccgaaacg aataaagtct ccccagctcc 5700

tgctgtgtag gctgggcaga aaccacaaaa aaaaaaaaaa aaaaaaa 5747

<210> 2
<211> 365
<212> PRT
<213> Homo sapiens

<300>
<308> NCBI/NP_055724
<309> 2004-08-23

<400> 2

Met Pro Gly Gly Lys Lys Val Ala Gly Gly Gly Ser Ser Gly Ala Thr
1               5                   10                  15

Pro Thr Ser Ala Ala Ala Thr Ala Pro Ser Gly Val Arg Arg Leu Glu
            20                  25                  30

Thr Ser Glu Gly Thr Ser Ala Gln Arg Asp Glu Glu Pro Glu Glu Glu
        35                  40                  45

Gly Glu Glu Asp Leu Arg Asp Gly Gly Val Pro Phe Phe Val Asn Arg
    50                  55                  60

Gly Gly Leu Pro Val Asp Glu Ala Thr Trp Glu Arg Met Trp Lys His
65                  70                  75                  80

Val Ala Lys Ile His Pro Asp Gly Glu Lys Val Ala Gln Arg Ile Arg
                85                  90                  95

Gly Ala Thr Asp Leu Pro Lys Ile Pro Ile Pro Ser Val Pro Thr Phe
            100                 105                 110

Gln Pro Ser Thr Pro Val Pro Glu Arg Leu Glu Ala Val Gln Arg Tyr
        115                 120                 125

Ile Arg Glu Leu Gln Tyr Asn His Thr Gly Thr Gln Phe Phe Glu Ile
    130                 135                 140

Lys Lys Ser Arg Pro Leu Thr Gly Leu Met Asp Leu Ala Lys Glu Met
145                 150                 155                 160

Thr Lys Glu Ala Leu Pro Ile Lys Cys Leu Glu Ala Val Ile Leu Gly
                165                 170                 175

Ile Tyr Leu Thr Asn Ser Met Pro Thr Leu Glu Arg Phe Pro Ile Ser
            180                 185                 190

Phe Lys Thr Tyr Phe Ser Gly Asn Tyr Phe Arg His Ile Val Leu Gly
        195                 200                 205

Val Asn Phe Ala Gly Arg Tyr Gly Ala Leu Gly Met Ser Arg Arg Glu
    210                 215                 220

Asp Leu Met Tyr Lys Pro Pro Ala Phe Arg Thr Leu Ser Glu Leu Val
225                 230                 235                 240

Leu Asp Phe Glu Ala Ala Tyr Gly Arg Cys Trp His Val Leu Lys Lys
                245                 250                 255

Val Lys Leu Gly Gln Ser Val Ser His Asp Pro His Ser Val Glu Gln
            260                 265                 270

Ile Glu Trp Lys His Ser Val Leu Asp Val Glu Arg Leu Gly Arg Asp
        275                 280                 285

Asp Phe Arg Lys Glu Leu Glu Arg His Ala Arg Asp Met Arg Leu Lys
    290                 295                 300

Ile Gly Lys Gly Thr Gly Pro Pro Ser Pro Thr Lys Asp Arg Lys Lys
305                 310                 315                 320

Asp Val Ser Ser Pro Gln Arg Ala Gln Ser Ser Pro His Arg Arg Asn
                325                 330                 335

Ser Arg Ser Glu Arg Arg Pro Ser Gly Asp Lys Lys Thr Ser Glu Pro
            340                 345                 350

Lys Ala Met Pro Asp Leu Asn Gly Tyr Gln Ile Arg Val
        355                 360                 365

## Description

### Field of the Invention

The present invention relates to a polypeptide having an anti-angiogenic activity, or a derivative thereof, or a pharmaceutically acceptable salt thereof or a solvate thereof, or a pharmaceutical composition containing the polynucleotide, or a derivative thereof, or a pharmaceutically acceptable salt thereof or a solvate thereof. In addition, the present invention relates to a method of screening various substances using the polypeptide.

### Background Art

In order that a cell is alive in a living body, oxygen and a nutrient which are supplied by blood are necessary, and angiogenesis is prevailing in a tissue in which proliferation is prevailing. It is known that angiogenesis is involved in not only regeneration of a tissue physiologically, but also a disease in which cell proliferation is prevailing pathologically. As the disease, a vascular disease, an inflammatory disease, an entoptic neovascular disease, a reproductive disease, a central nervous system disease or a cancer is reported (Non-Patent Document 1). Furthermore, since angiogenesis is seen at proliferation of a malignant neoplasm cell, a human umbilical vein endothelial cell (HUVEC) treated with vascular endothelial growth factor (VEGF) is reported as a model of angiogenesis by a cancer (Non-Patent Document 2).

Genes involved in the aforementioned various diseases have been previously studied, but a sideration mechanism thereof has not been completely elucidated, and possibility of involvement of an unknown gene is considered. Under such the circumstances, identification of a novel gene involved in sideration of the diseases, and a method of diagnosing a disease using the gene have been desired.

Thereafter, as a polypeptide having an anti-angiogenic activity, a polypeptide consisting of 365 amino acids described in SEQ ID No. :2 (hereinafter, referred to as vasohibin) has been found out (Patent Document 1 and Non-Patent Document 3). A nucleotide sequence (SEQ ID No. :1) of a cDNA encoding the polypeptide is disclosed as KIAA1036 in Non-Patent Document 4.
[Patent Document 1] WO 02/090546
[Non-Patent Document 1] Nature, 407, 249(2000)
[Non-Patent Document 2] Cancer Research, 55, 5296-5301(1995)
[Non-Patent Document 3] The Journal of Clinical Investigation, 114(7) 898-907(2004)
[Non-Patent Document 4] DNA Research, 6(3) 197-205(1999)
[Non-Patent Document 5] Biochemical and Biophysical Research Communications, 327, 700-706(2005)

### Disclosure of Invention

### Problems to be solved by the Invention

Although vasohibin has been known to have an anti-angiogenic activity, a polypeptide having an anti-angiogenic activity, which is suitable as an active ingredient of a medicament and has a lower molecular weight, has been desired.

### Means for Solving the Problem

The present inventors intensively studied and, as a result, found out four kinds of fragments (42 kD, 36 kD, 32 kD, 27 kD), by forcibly expressing vasohibin (44 kD) in a vascular endothelial cell. Furthermore, the present inventors analyzed those fragments and, as a result, found out that a sequence necessary for exerting an anti-angiogenic activity is an amino acid sequence on a C terminus side from arginine at a 318-position described in SEQ ID No.:2, that is, an amino acid sequence of a 319-position to a 365-position.

According to the present invention, it has become possible to provide a polypeptide which has a lower molecular weight than that of vasohibin and has an anti-angiogenic activity. Low molecular weight vasohibin provided in the present invention has an anti-angiogenic activity like vasohibin, and is useful as a therapeutic agent for various diseases such as a vascular disease associated with angiogenesis, an inflammatory disease, an entoptic neovascular disease, a reproductive disease, a central nervous system disease and cancer and the like. Usually, when a protein preparation is prepared, it is desirable that a polypeptide which is an active ingredient has a lower molecular weight, and the low molecular weight vasohibin of the present invention is very useful. In addition, the low molecular weight vasohibin is also useful as a marker for angiogenesis.

This invention is
(1) a polypeptide consisting of continuous not more than 364 amino acids of an amino acid sequence represented by Met at a 1-position to Val at a 365-position of SEQ ID No. 2, and comprising an amino acid sequence of Lys at a 319-position to Val at a 365-position, or a derivative thereof, or a pharmaceutically acceptable salt thereof or a solvate thereof,
(2) the polypeptide according to (1), wherein the polypeptide comprises an amino acid sequence of Met at a 77-position to Val at a 365-position, or a derivative thereof, or a pharmaceutically acceptable salt thereof or a solvate thereof,
(3) the polypeptide according to (1), wherein the polypeptide comprises an amino acid sequence of Arg at a 30-position to Val at a 365-position, or a derivative thereof, or a pharmaceutically acceptable salt thereof or a solvate thereof,
(4) the polypeptide according to (1), wherein the polypeptide consists of an amino acid sequence starting at any amino acid of Arg at a 30-position to Arg at a 318-position and terminating at Val at a 365-position, or a derivative thereof, or a pharmaceutically acceptable salt thereof or a solvate thereof,
(5) the polypeptide according to (1), wherein the polypeptide consists of an amino acid sequence starting at any amino acid of Met at a 77-position to Agr at a 318-position and terminating at Val at a 365-position, or a derivative thereof, or a pharmaceutically acceptable salt thereof or a solvate thereof,
(6) the polypeptide according to (1), wherein the polypeptide consists of an amino acid sequence starting at any amino acid of Arg at a 30-position to Met at a 77-position and terminating at Val at a 365-position, or a derivative thereof, or a pharmaceutically acceptable salt thereof or a solvate thereof,
(7) a polypeptide in which one or a few amino acid residues are deleted or substituted in the polypeptide as defined in any one of (1) to (6), wherein the polypeptide has an anti-angiogenic activity, or a derivative thereof, or a pharmaceutically acceptable salt thereof or a solvate thereof,
(8) a method of screening a substance which binds to the polypeptide as defined in any one of (1) to (7), comprising the following;
   (a) a step of contacting the polypeptide as defined in any one of (1) to (7) with a test substance, and
   (b) a step of detecting binding of the polypeptide with the test substance,
(9) a method of screening a binding activity regulating substance which regulates binding of the polypeptide as defined in any one of (1) to (7) with a substance which binds to the polypeptide, comprising the following;
   (a) a step of contacting the polypeptide as defined in any one of (1) to (7) with a binding substance of the polypeptide in the presence or the absence of a test substance, and
   (b) a step of comparing activity of binding of the polypeptide with the binding substance between in the presence and in the absence of the test substance,
(10) a method of screening a substance which regulates expression of the polypeptide as defined in any one of (1) to (7), comprising the following;
   (a) a step of a cell which can express the polypeptide as defined in any one of (1) to (7) with a test substance, and
   (b) a step of detecting a change in an expression amount of the polypeptide,
(11) a pharmaceutical composition comprising the polypeptide as defined in any one of (1) to (7), or a derivative thereof, or a pharmaceutically acceptable salt thereof or a solvate thereof,
(12) the pharmaceutical composition according to (11), which is a therapeutic agent for a vascular disease, an inflammatory disease, an entoptic neovascular disease, a reproductive system disease, a central nervous system disease or cancer.

Since the polypeptide of the present invention, or a derivative thereof, or a pharmaceutical acceptable salt thereof or a solvate thereof has an anti-angiogenic activity, and has a lower molecular weight as compared with vasohibin, it is very useful as an active ingredient used in a pharmaceutical composition. In addition, by using the polypeptide of the present invention, a substance binding to the polypeptide of the present invention can be screened. A compound obtained by the screening can be used as an anti-angiogenic agent or an angiogenesis inducing agent.

### Brief Description of the Drawings

[Fig.1] Inhibition of processing of 42 kD vasohibin protein by Arg29Ala substitution
[Fig.2] Inhibition of processing of 36 kD vasohibin protein by Arg76Ala substitution
[Fig.3] Inhibition of processing of 32 kD and 27 kD vasohibin proteins by Arg76Ala substitution
[Fig.4] Processing pattern of vasohibin protein
[Fig.5] Inhibition effect of vasohibin (77-365) protein on in vivo angiogenesis
[Fig.6] Inhibition effect of vasohibin (77-318) protein on in vivo angiogenesis

### Best Mode for Carrying Out the Invention

Terms used herein are used as having the meaning which is usually used in the art unless otherwise indicated. Terms particularly used herein will be explained below.

"Angiogenesis" means phenomenon in which a vascular endothelial cell is budded from the existing vascular vessel, and a capillary vessel is formed in a form of entrance into a tissue. A formation process progresses in an order of 1) digestion of a vascular basal membrane with a protease, 2) wandering/proliferation of a vascular endothelial cell, and 3) formation of a lumen.

Examples of a disease associated with "angiogenesis" include a "vascular disease", an "inflammatory disease", an "entoptic neovascular disease", a "reproductive disease", a "central nervous system disease", "cancer" and the like.

Examples of the "vascular disease" include arteriosclerosis, hypertension, angina, constructive arteriosclerosis, myocardial infarction, brain infarction, diabetic vascular disorder, blood vessel malformation and the like.

Examples of the "inflammatory disease" include hepatitis, pneumonia, glomerular nephritis, thyroiditis, myelitis, synovitis, bone destruction, cartilage destruction, rheumatoid, asthma, sarcoidosis, Crow-Fukase syndrome, pannus, allergic edema, ulcer, ascites, peritoneal sclerosis, tissue adhesion and the like.

Examples of the "entoptic neovascular disease" include diabetic retinopathy, retinal vein obstruction, and aging macular degeneration and the like.

Examples of the "reproductive disease" include uterine dysfunction, placenta dysfunction, ovary hyperergasia, follicular cyst and the like.

Examples of the "central nervous system disease" include retinopathy, cerebral apoplexy, vascular dementia, Alzheimer's disease.

Examples of the "cancer" include various malignant neoplasms such as solid cancer, angioma, hemangioendotielioma, sarcoma, Kaposi sarcoma and hematopoietic tumor, include ovary cancer and large intestine cancer and, further, include metastasis of these cancers.

The "polypeptide of the present invention" includes:
(1) a polypeptide consisting of continuous not more than 364 amino acids of an amino acid sequence represented by Met at a 1-position to Val at a 365-position of SEQ ID No.:2, and comprising an amino acid sequence of Lys at a 319-position to Val at a 356-position;
(2) the polypeptide according to (1), wherein the polypeptide comprises an amino acid sequence of Met at a 77-position to Val at a 365-position;
(3) the polypeptide according to (1), wherein the polypeptide comprises an amino acid sequence of Arg at a 30-position to Val at a 365-position;
(4) the polypeptide according to (1), wherein the polypeptide consists of an amino acid sequence starting at any amino acid of Arg at a 30-position to Arg at a 318-position and terminating at Val at a 365-position;
(5) the polypeptide according to (1), wherein the polypeptide consists of an amino acid sequence starting at any amino acid of Met at a 77-position to Arg at a 318-position and terminating at Val at a 365-position;
(6) the polypeptide according to (1), wherein the polypeptide consists of an amino acid sequence starting at any amino acid of Arg at a 30-position to Met at a 77-position and terminating at Val at a 365-position.

That is, examples of the polypeptide of the present invention include a polypeptide consisting of continuous not more than 364 amino acids of an amino acid sequence represented by Met at a 1-position to Val at a 365-position of SEQ ID No.:2. In addition, examples include a polypeptide comprising a continuous amino acid sequence of a 319-position to a 365-position. For examples, aspects of (4), (5) and (6) are preferable. Particularly, an aspect of (6) is preferable. All of the aforementioned polypeptides of the present invention have an anti-angiogenic activity. Specific examples include a polypeptide represented by an amino acid sequence starting at Arg at a 30-position and terminating at Val at a 365-position, a polypeptide represented by an amino acid sequence starting at Met at a 77-position and terminating at Val at a 365-position and the like.

The polypeptide of the present invention also includes a "polypeptide in which one or a few amino acid residues are deleted or substituted in the polypeptide described in any one of (1) to (6), and which has an anti-angiogenic activity".

One or a few amino acids is amino acids at such an extent of the number that can be deleted or substituted by site-specific mutagenesis or the like, and means 1 to 50, preferably 1 to 30, more preferably 1 to 20, further preferably 1 to 10, further preferably 1 to 5, further preferably 1, 2 or 3 amino acid residues. One or a few amino acids even derived from deletion or substitution is included in the polypeptide of the present invention as far as an anti-angiogenic activity is possessed.

The "anti-angiogenic activity" of the polypeptide of the present invention can be measured by the known method. For example, the activity can be measured by the method described in Example 3 described later.

The "derivative" of the polypeptide of the present invention means a polypeptide having substantially the same biological function or activity as that of the polypeptide of the present invention, that is, an anti-angiogenic activity. A C-terminus of the derivative of the polypeptide may be any of a carboxyl group, carboxylate, amide and ester. Examples include a modified polypeptide obtained by adding a modification group, a mutant polypeptide obtained by altering an amino acid residue and the like.
Examples of the modification group include a functional group exhibiting the fluorescence property, and a functional group which is not involved in formation of a steric structure of a polypeptide. Alternatively, the modification group may be an amino acid residue other than amino acid residues constituting a nature-derived polypeptide of the present invention (e.g. β-alanine etc.). Examples of the functional group exhibiting the fluorescence property include eosin and fluorescein isothiocyanate (FITC). Examples of the functional group which is not involved in formation of a steric structure of a polypeptide include a spacer group, a representative which is typified by a β-alanine residue, etc. It is preferable that such the functional group is present at a terminus.

Examples of the "pharmaceutically acceptable salt" of the polypeptide of the present invention include a physiologically acceptable salt with an acid or a base, and an acid addition salt is particularly preferable. As such the salt, salts with inorganic acids such as hydrochloric acid, phosphoric acid, hydrobromic acid, sulfuric acid and the like, or salts with organic acids such as acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like are used.

The "solvate" of the present polypeptide or the aforementioned salt may be such that the polypeptide of the present invention is coordinated with an arbitrary number of solvent molecules. A preferable is a hydrate.

The present invention relates to a method of screening a substance which binds to the polypeptide of the present invention. The "substance which binds" is an arbitrary substance which binds to the polypeptide of the present invention, and examples of such the binding substance include a low-molecular substance, a polypeptide such as a receptor and an antibody and the like. The method of screening a binding substance can be performed by appropriate applying the technique which is known in the art, and examples include bioassay and binding assay.

The present invention further relates to a "method of screening a binding activity regulating substance". The "binding activity regulating" means increase or decrease in the activity of binding of the polypeptide of the present invention with a substance which binds thereto, and the "binding activity" is assessed by Percent Maximum Binding (PMB). The "binding activity regulating substance" is a substance which increases or decreases the activity of binding of the polypeptide of the present invention with a substance which binds thereto, and examples include an agonist and an antagonist. Examples of such the substance include a low-molecular substance, a polypeptide and the like. The "method of screening a binding activity regulating substance" can be accomplished by appropriately applying the technique which is known in the art. By the screening method of the present invention, the binding activity regulating substance can be screened, and examples of such the method include bioassay and binging assay.

Also, the present invention relates to a "method of screening an expression regulating substance". An expression amount means an amount of expression of the polypeptide of the present invention in an objective cell or the like. An expression amount can be assessed by measurement by an appropriate method including an immunological measuring method such as an ELISA method, an IRA method, a fluorescent antibody method, an immunohistological staining method and the like using an antibody to the polypeptide of the present invention. The "expression regulation" means increase or decrease in an expression amount of the polypeptide of the present invention at a protein level, which is assessed by an arbitrary appropriate method including the immunological measuring method. The "expression regulating substance" is a substance which increases or diseases an expression amount of the polypeptide of the present invention at a protein level, which is assessed by an arbitrary appropriate method including the immunological measuring method and examples of such the substance include a low-molecular substance, a polypeptide and the like. The "method of screening an expression regulating substance" can be attained by appropriately applying the technique which is known in the art. By the screening method of the present invention, the expression regulating substance can be screened, and examples of such the method include bioassay and binding assay.

As the "pharmaceutical composition" of the present invention, it is only necessary to contain at least the polypeptide of the present invention, or a pharmaceutically acceptable salt thereof or a solvate thereof, and can be used in a therapeutic agent such as a vascular disease, an inflammatory disease, an entoptic neovascular disease, a reproductive disease, a central nervous system disease and cancer and the like. In addition, the polypeptide of the present invention may be PEGylated or glycosylated.

The "treating method" is a method of treating a particular disease, for example, a vascular disease, an inflammatory disease, an entoptic neovascular disease, a reproductive disease, a central nervous system disease and cancer and the like which is a disease associated with angiogenesis by administering the pharmaceutical composition described above.

In addition, the polypeptide of the present invention can be a marker for angiogenesis and various diseases associated therewith. Herein, the "marker" means a substance used for detecting or quantitating angiogenesis and various diseases associated with therewith, for examples, a vascular disease, an inflammatory disease, an entoptic neovascular disease, a reproductive disease, a central nervous system disease and cancer and the like, from a biological sample.

The "detection or quantitation" of the polypeptide of the present invention can be attained using an arbitrary appropriate method including an immunological measuring method.

When the polypeptide of the present invention is used as a marker of angiogenesis and various diseases associated therewith, examples of a method of detecting or quantitating the polypeptide of the present invention include an immunological measuring method such as an ELISA (Enzyme Linked Immuno Sorbent Assay) method, a RIA (Radio Immuno Assay) method, a fluorescent antibody method, a Western blotting method, an immunohistological staining method and the like.

Using an antibody to the polypeptide of the present invention or a fragment thereof, angiogenesis, a vascular disease, an inflammatory disease, an entoptic neovascular disease, a reproductive disease, a central nervous system disease or cancer can be detected or quantitated. Since the polypeptide of the present invention can be a marker of the aforementioned diseases, the method of detecting or quantitating a disease using an antibody can be attained by the method including suitable immunological measuring methods like the aforementioned detection or quantitation of the polypeptide.

The present invention includes a kit for diagnosing a vascular disease, an inflammatory disease, an entoptic neovascular disease, a reproductive disease, a central nervous system disease or cancer comprising the polypeptide of the present invention as a standard reagent. This kit may further contain an antibody to the polypeptide of the present invention or a fragment thereof. Since the polypeptide of the present invention is a marker of angiogenesis, a vascular disease, an inflammatory disease, an entoptic neovascular disease, a reproductive disease, a central nervous system disease or cancer, the disease can be measured immunologically based on the aforementioned detection or quantitation method by an immunological measuring method.

Also, the present invention includes:
(I) an expression vector for inhibiting angiogenesis, comprising a polynucleotide encoding the polypeptide of the present invention, and
(II) an expression vector for inducing angiogenesis comprising a polynucleotide which hybridizes with a polynucleotide encoding the polypeptide of the present invention under the stringent condition, and encodes a polypeptide having an anti-angiogenic activity. These are hereinafter referred to as "present vector" in some cases.

The "polynucleotide which hybridizes under the stringent condition" means a polynucleotide obtained by employing a fragment of a polynucleotide encoding the polypeptide of the present invention as a probe, and using the procedure which is well known and is conventional in the art, for example, a colony hybridization method, a plaque hybridization method or a Southern blotting hybridization method, specifically means a polynucleotide which can be identified by performing hybridization at 65°C in the presence of 0.7 to 1.0 mM NaCl using a membrane with a polynucleotide derived from a colony or plaque immobilized thereon, thereafter, washing the membrane at 65°C using SSC (Saline Sodium Citrate; 150 mM sodium chloride, 15 mM sodium citrate) having a 0.1 to 2-fold concentration. Hybridization can be performed according to the method described in Molecular Cloning: A Laboratory Manual, Second Edition (1989) (Cold Spring Harbor Laboratory Press), Current Protocols in Molecular Biology (1994) (Wiley-Interscience), DNA Cloning 1:Core Techniques, Practical Approach Second Edition (1995) (Oxford University Press) and the like.

Herein, the "polynucleotide which hybridizes" refers to a polynucleotide which can hybridize with another polynucleotide under the aforementioned hybridization condition. Examples of such the polynucleotide include a polynucleotide having at least not lower than 60% homology, preferably a polynucleotide having not lower than 80% homology, further preferably a polynucleotide having not lower than 95% homology with a nucleotide sequence of a DNA encoding the polypeptide of the present invention. Herein, for homology, similarity is indicated by a score, for example, using a retrieving program BLAST employing an algorism developed by Altschul et al. (The Journal of Molecular Biology, 215, 403-410(1990)).

The present invention includes an angiogenesis inducing agent containing a neutralizing antibody to the polypeptide of the present invention or a salt thereof, or an aptamer to the polypeptide of the present invention.

The "antibody" is used as having the meaning which is usually used in the art, and includes a whole antibody or a fragment thereof, a derivative, a bound body, a modified body and the like. Preferably, the antibody is an antibody recognizing the polypeptide of the present invention or a fragment thereof, more preferably an antibody specifically recognizing the polypeptide, further preferably an antibody mono-specifically recognizing the polypeptide. Such the antibody may be any of a polyclonal antibody and a monoclonal antibody.

The "aptamer" means a nucleic acid molecule having the ability to specifically bind to a target protein. The aptamer may be a RMA or a DNA, and may be single-stranded or double-stranded as far as it specifically binds to the polypeptide of the present invention. A length of bases of the aptamer is not particularly limited as far as the aptamer has a necessary length for specifically binding to the polypeptide of the present invention, but the length is ,for example, 10 to 200 bases, preferably 20 to 150 bases, more preferably, 30 to 150 bases, further preferably 50 to 150 bases.
The aptamer can be obtained, for example, by an in vitro selection method. The "in vitro selection method" is a method of obtaining a nucleic acid molecule having the particular function, by repeatedly performing a selection process of separating a single-stranded oligonucleotide having the particular function (e.g. ability to be specifically adsorbed on a target substance) from a randomly synthesized single-stranded oligonucleotide library, amplifying this and, further, separating a single-stranded oligonucleotide having the particular function. When one wants to obtain the aptamer from a random oligonucleotide library, examples of a method of separating a nucleic acid molecule having the adsorption ability include affinity chromatography using an affinity column on which a target substance is immobilized.

The "anti-angiogenic agent" has the action of inhibiting angiogenesis by utilizing or promoting the action of the polypeptide of the present invention having the novel anti-angiogenic activity. Examples of the agent include an agent containing the polypeptide of the present invention, a polypeptide encoding the polypeptide or the like, and an agent containing a compound having the action of promoting an anti-angiogenic activity of the polypeptide, obtained by screening using the polypeptide of the present invention, or a salt thereof, and the like (hereinafter, referred to as "present anti-angiogenic agent" in some cases). The anti-angiogenic agent can be used as an agent for preventing or treating a disease which can have the serious condition by occurrence of angiogenesis among the aforementioned diseases. Examples of a disease for which the anti-angiogenic agent is effective include a vascular disease, an inflammatory disease, an entoptic neovascular disease, a reproductive disease, a central nervous system disease and a malignant neoplasm.

The "angiogenesis inducing agent" has the action of inducing angiogenesis by inhibiting the action of the polypeptide of the present invention having the novel anti-angiogenic activity. Examples of the agent include an agent containing a neutralizing antibody or a aptamer recognizing the polypeptide of the present invention or a polynucleotide encoding the polypeptide, and an agent containing a compound having the action of inhibiting an anti-angiogenic activity of the polypeptide, obtained by screening using the polypeptide of the present invention, or a salt thereof (hereinafter, referred to as "present angiogenesis inducing agent" in some cases). The angiogenesis inducing agent can be used as an agent for preventing or treating a disease, the condition of which can be alleviated by occurrence of angiogenesis among the aforementioned diseases. Examples of a disease for which the angiogenesis inducing agent is effective include curing of damage, restoration of inflammation, ischemic heart disease and peripheral vascular disease, arteriosclerosis and the like.

A method of obtaining a polynucleotide encoding the polypeptide of the present invention (hereinafter, referred to as "present gene" in some cases), a method of preparing the polypeptide of the present invention, a method of screening a substance which binds to the polypeptide, a method of screening a binding activity regulating substance which regulates binding of the polypeptide with a substance which binds to the polypeptide, and a method of screening a substance which regulates expression of the polypeptide will be explained in detail below.

Herein, unless otherwise indicated, the technique of gene recombination, the technique of production of a recombinant polypeptide with an animal cell, an insect cell, yeast and Escherichia coli, a molecular biological procedure, a method of separating and purifying an expressed polypeptide, an analysis method and an immunological procedure which are known in the art can be adopted.

### Method of obtaining present gene

A DNA encoding vasohibin is obtained by preparing a cDNA library from a human brain, heart, skeletal muscle, spleen, kidney, liver, small intestine, placenta, a human normal cell delivered from these tissues, or a human umbilical vein endothelial cell by a conventional method. In addition, a DNA encoding objective vasohibin can be also prepared by performing PCR using an oligonucleotide as a sense primer and an anti-sense primer and employing, as a template, a cDNA prepared from a mRNA of a cell expressing a mRNA complementary with these DNAs. The thus obtained DNA encoding vasohibin is cut with an appropriate restriction enzyme, thereby, a DNA encoding the polypeptide of the present invention can be obtained.

Examples of a method of preparing a cDNA library include the method described in Molecular Cloning: A Laboratory Manual, Second Edition (1989) (Cold Spring Harbor Laboratory Press), Current Protocols in Molecular Biology (1994)(Wiley-Interscience), DNA Cloning 1: Core Techniques, A Practical Approach, Second Edition (1995)(Oxford University Press) and the like, and a method using a commercially available kit, for example, SuperScript Plasmid System for cDNA Synthesis and Plasmid cloning (manufactured by Invitrogen) and ZAP-cDNA synthesis Kits (manufactured by Stratagene).

Alternatively, a polynucleotide encoding the polypeptide of the present invention can be prepared by chemically synthesizing a DNA encoding the polypeptide of the present invention based on an amino acid sequence. Chemical synthesis of a DNA can be performed using a DNA synthesizer manufactured by Shimadzu Corporation utilizing a thiophosphite method, a DNA synthesizer model1392 manufactured by Perkin Elmer utilizing a phosphoamidite method, or the like.

### Preparation of polypeptide of the present invention

The polypeptide of the present invention can be prepared by expressing a polynucleotide encoding the polypeptide of the present invention in a host cell, for example, by the following method using the method described in Molecular Cloning: A Laboratory Manual Second Edition (1989) (Cold Spring Harbor Laboratory Press), Current Protocols in Molecular Biology (1994)(Wiley-Interscience), or the like.
If necessary, a DNA fragment having a suitable length containing a part encoding the polypeptide of the present invention is prepared based on a full length DNA encoding vasohibin. In addition, a DNA in which a base in a nucleotide sequence of a part encoding the polypeptide is substituted so as to be a codon optimal for expression of a host. The DNA is useful for improving productivity of the polypeptide. By inserting the DNA fragment or a full length DNA downstream of a promoter of a suitable expression vector, a recombinant DNA (recombinant vector) is prepared. By introducing the recombinant vector into a host cell suitable for the expression vector, a transformant producing the polypeptide of the present invention can be obtained.
As a host cell, any cell such as a prokaryotic cell, yeast, an animal cell, a plant cell, an insect cell, and the like can be used as far as it can express an objective gene. As an expression vector, a vector which can be self-replicated in the host cell, or can be incorporated into a chromosome, and has a promoter at a suitable position for transcribing a gene of the polypeptide of the present invention is used.

(i) Case where prokaryote is used as host
It is preferable that an expression vector of the polypeptide of the present invention can be self-replicated in a prokaryote and, at the same time, is constructed of a promoter, a ribosome binding sequence, a DNA encoding the polypeptide of the present invention, and a transcription termination sequence. A gene controlling a promoter may be contained.

Examples of the expression vector include pBTrp2, pBTacl, pBTac2 (manufactured by Roche Diagnostics), Bluescript II SK (+), pBluescript II SK(-) (manufactured by Stratagene), pSTV28, pUC118, pUC19 (manufactured by TAKARA SHUZO Co., Ltd.), pKK233-2 (manufactured by Pharmacia), pSE280, pSupex, pUB110, pTP5, pC194, pTrxFus (manufactured by Invitrogen), pGEMEX-1 (manufactured by Promega), pQE-8 (manufactured by Qiagen), pGEX (manufactured by Pharmacia), pET system (manufactured by Novagen), pMAL-c2 (manufactured by New England Biolabs), pKYP10 (JP-A No.58-110600), pXYP200 (Agricultural Biological Chemistry, 48, 669(1984)), pLSA1 (Agricultural Biological Chemistry), 53, 277(1989)), pGEL1 (Proceeding of the National Academy of Sciences USA, 82,4306(1985)), pEG400 (Journal of Bacteriology, 172, 2392(1990)), pTrs30(FERM BP-5407), pTrs32 (FERM BP-5408), pGHA2 (FERM BP-400), pGKA2 (FERM B-6798), pPA1 (JP-A No.63-233798), pTerm2 (JP=A No.3-22979, US4686191, US4939094, US5160735) and the like.

As the promoter, any promoter can be used as far as expression is possible in a host cell such as Escherichia coli and the like. Examples include a promoter derived from Escherichia coli and phage such as a trp promoter (Ptrt), a lac promoter (Plac), a PL promoter, a PR promoter, a PSE promoter and the like, a SPO1 promoter, a SOP2 promoter, a penP promoter and the like. In addition, an artificially designed and modified promoter such as a promoter in which two Ptrps are connected in series (Ptrpx2), a tac promoter, lacT7 promoter, and a letI promoter can be also used.

It is preferable to use a plasmid in which a distance between a Shine-Dalgarno sequence which is a ribosome binding sequence, and an initiation codon is suitably regulated, for example, is regulated at 6 to 18 bases. Although a transcription termination sequence is not necessarily required for expression of the polynucleotide of the present invention, it is preferable to dispose a transcription termination sequence immediately downstream of a structural gene.

Examples of the host cell include a prokaryote such as genus Escherichia, genus Serratia, genus Bacillus, genus Brevibacterium, genus Corynebacterium, genus Microbacterium, genus Pseudomonas and the like, examples of the genus Escherichia include XL1-Blue strain, XL2-Blue strain, DH1 strain, MC1000 strain, KY3276 strain, W1485 strain, JM109 strain, HB101 strain, No.49 strain, W3110 strain, NY49 strain, BL21 (DE3) strain, BL21 (DE3) pLysS strain, HMS174(DE3) strain and HMS174(DE3) pLysS strain of E. coli, examples of the genus Serratia include S. ficaria strain, S. fonticola strain, S. liquefaciens, S. marcescens strain and the like, examples of the genus Bacillus include B. subtilis stain, B. amyloliquefaciens strain, and the like, examples of the genus Brevibacterium include B. ammoniagenes strain, B. Immariophilum (ATCC:14068) strain, B. saccharolyticum (ATCC:14066) strain and the like, examples of the genus Corynebacterium include C. glutamicum (ATCC:13032) strain, C. glutamicum (ATCC:14067) strain, C. gulutamicum (ATCC:13869) strain, C. acetoacidophilum (ATCC:13870) strain and the like, examples of the genus Microbacterium include M. ammoniaphilum (ATCC:15354) strain and the like, and examples of the genus Pseudomonas include S. mephitica strain and the like.

As the method introducing a recombinant vector, any method can be used as far it is a method of introducing a DNA into a host cell, and examples include an electroporation method (Nucleic Acids Research, 16, 6127(1988)), a calcium phosphate method (Proceedings of the National Academy of Sciences USA, 69, 2110(1972)), a protoplast method (JP-A No. 63-2148942), a method described in Gene, 17, 107(1982) and Molecular & General Genetics, 168, 111(1979), and the like.

(ii) Case where yeast is used as host
When yeast is used as a host, examples of the expression vector include YEp13 (ATCC:37115), YEp24 (ATCC:37051), YCp50 (ATCC:37419), pHS19, pHS15 and the like.
As the promoter, any promoter can be used as far as expression is possible in yeast, and examples include ADH1 (alcohol dehydrogenase) promoter , PHO5 (acidic phosphatase) promoter, PGK1 (phosphoglyceric acid kinase) promoter, GAPDH (glyceraldehyde-3-phosphate dehydrogenase) promoter, GAL1 (galactose kinase) promoter, GAL10 (UDP galactose 4-epimerase) promoter, MFα1 (α pheromone) promoter, CUP 1 (metallothionein) promoter and the like.
Examples of the host include genus Saccharomyces, species S. cerevisiae, genus Schizosaccharomyces, species, S. pombe, genus Kluyveromyces, species K.lactis, genus Trichosporon, species T. pullulans, genus Schwanniomyces, species S. alluvius and genus Pichia, species P. pastoris and the like.
As the method of introducing a recombinant vector, any method can be used as far as it is a method of introducing a DNA into a host, and examples include electroporation method (Methods in Enzymology, 194, 182(1990)), a spheroplast method (Proceedings of the National Academy of Sciences USA, 84, 1929(1978)), a lithium acetate method (Journal of Bacteriology,153, 163(1983)) and the method described in Proceedings of the National Academy of Sciences USA, 75, 1929(1978)).

(iii) Case where animal cell is used as host
When an animal cell is used as a host, as an expression vector, for example, pcDNA1/Amp, pcDNA1, pCDM8, pREP4 (manufactured by Invitrogen), pAGE107 (Cytotechnology, 3, 133 (1990)), pAGE103 (The Journal of Biochemistry, 101, 1307 (1987)), pAMo, pAMoA (pAmoPRSA) (The Journal of Biological Chemistry, 268, 22782-22787 (1993)), pAS3-3 (JP-A No. 2-22705) and the like can be used.
As the promoter, any promoter can be used as far as expression is possible in a host, and examples include a promoter of IE (immediate-early) gene of human cytomegalovirus (hCMV), an early promoter of SV40, a long terminal repeat promoter of Moloney murine leukemia virus, a promoter of a retrovirus, a HSP promoter, a SR α promoter and a metallothionein promoter. Alternatively, an enhancer of a human CMV IE gene may be used together with a promoter.

As the animal cell used in a host cell, there can be exemplified a human-derived strain cell HEK293 (human fetal kidney cell, ATCC:CRL-1573), Namalwa (Burkitt's lymphoma, ATCC:CRL-1432), Hela (uterine cervical cancer cell, ATCC:CCL-2), HBT5637 (leukemia cell, JP-A No. 63-299), BALL-1 (leukemia cell) and HCT-15 (large intestine cancer cell), a mouse-derived strain cell Sp2/0-Ag14 (mouse myeloma cell, ATCC:CRL-1581) and NSO (mouse myeloma cell), a monkey-derived strain cell COS-1 (African green monkey kidney cell (SV40 transformant cell), ATCC:CRL-1650) and COS-7 (African green monkey kidney cell (SV40 transformant cell), ATCC:CRL-1651), a hamster-derived strain cell CHO-K1 (Chinese hamster ovary cell, ATCC:CCL-61) and BHK-21 (C-13) (Sicilian hamster offspring kidney cell, ATCC:CCL-10), a rat-derived strain cell PC12 (adrenal brown cell tumor, ATCC:CRL-1721) and YB2/0 (rat myeloma cell, ATCC:CRL-1662) and the like.

As the method of introducing a recombinant vector, any method can be used as far as it is a method of introducing a DNA, and examples include an electroporation method (Cytotechnology, 3, 133, (1990)), a calcium phosphate method (JP-A No. 2-22705), a lipofection method (Proceedings of the National Academy of Sciences, USA, 84, 7413 (1987), Virology, 52, 456 (1973)).

(iv) Case where plant cell is used as host
When a plant cell or a plant individual is used as a host cell, a polypeptide can be produced according to the known method (The Tissue Culture, 20 (1994), The Tissue Culture, 21 (1995), Trends in Biotechnology, 15, 45 (1997)). Examples of the expression vector include a Ti plasmid, a tobacco mosaic virus vector and the like. As a promoter used in gene expression, any promoter can be used as far as expression is possible in a plant cell, and examples include a 35S promoter of a cauliflower mosaic virus (CaMV), a rice actin 1 promoter and the like. In addition, by inserting intron 1 or the like of a corn alcohol dehydrogenase gene between a promoter and a gene to be expressed, the gene expression efficacy can be enhanced.

Examples of the host include cells of a plant such as potato, tobacco, corn, rice, rape, soy bean, tomato, carrot, wheat, barley, rye, alfalfa, flax and the like.
As the method of introducing a recombinant vector, any method can be used as far as it is a method of introducing a DNA into the host, and examples include a method using Agrobacterium (JP-A No. 59-140885, JP-A No. 60-700800, WO 94/00977), an electroporation method (JP-A No. 60-251887), a particle gun method (Japanese Patent No. 2606856, Japanese Patent No. 2517813) and the like.

(v) Case where insect cell is used as host
When an insect cell is used as the host, examples of a transfer vector include pv11392, pVL1393, pBlueBacIII (manufactured by Invitrogen) and the like, and examples of a virus for infecting include vaculovirus, Autographa california nuclear polyhedrosis virus (Acmnpv) Bac-N-Blue DNA, which infects a cabbage army warm family insect, and the like. As a method of transforming an insect cell, the method described in Baculovirus Expression Vector: A Laboratory Manual (1992) (W.H. Freeman and Company), Molecular Cloning: A Laboratory Manual, Second Edition (1989) (Cold Spring Harbor Laboratory Press), Current Protocols in Molecular Biology (1994) (Wiley-Intersicence), BioTechnology, 6, 47(1988) and the like may be used.

A polypeptide can be expressed by adding a transfer vector containing an objective gene and a baculovirus DNA for infecting an insect cell to an insect cell culturing solution, and infecting the insect cell with a virus expressing an objective gene prepared by recombination.
Examples of the insect cell used as the host include Spodoptera frugiperda (cabbage army warm)-derived strain cell, Trichoplusia ni (stinging nettle)-derived strain cell and the like and, specifically, examples of the S. frugiperda-derived cell include Sf9 (atcc:CRL-1711, ovary cell), Sf21 (ovary cell) and the like, and examples of the T. ni-derived cell strain include High Five, DTI-TN-5B1-4 (egg cell, manufactured by Invitrogen).
As a method of introducing a recombinant vector, any method can be used as far as it is the method which can introduce the vector into a host, and examples include a calcium phosphate method (JP-A No. 2-22705), a lipofection method (Proceedings of the National Academy of Sciences USA, 84, 7413 (1987)) and the like. Alternatively, like the animal cell, an electroporation method (Cytotechnology, 3, 133 (1990)) and the like can be used.

(vi) Culturing method
When a transformant harboring a recombinant vector with a DNA encoding the polypeptide of the present invention incorporated therein is a cell such as Escherichia coli, yeast, an animal cell and a plant cell, the polypeptide can be produced by culturing the cell according to a normal culturing method suitable for various hosts, producing and accumulating the polypeptide, and recovering the polypeptide from a transformant or a culturing solution. When a transformant is an animal individual or a plant individual, the polypeptide can be produced by rearing or cultivating the individual according to a normal growing method suitable for various hosts, producing and accumulating the polypeptide, and recovering the polypeptide from the animal individual or the plant individual.
When a host is an animal individual, the polypeptide of the present invention can be produced by rearing a non-human transgenic animal harboring the polynucleotide of the present invention, producing and accumulating the polypeptide of the present invention encoded by the recombinant DNA in the animal, and recovering the polypeptide from the animal individual. Examples of a place of production and accumulation in an animal individual include a milk, a saliva, an egg and the like of the animal.
When the host is a plant individual, the polypeptide of the present invention can be produced, for example, by cultivating a transgenic plant harboring a polynucleotide encoding the polypeptide of the present invention, producing and a accumulating the polypeptide of the present invention encoded by the recombinant DNA in the plant individual, and recovering the polypeptide from the plant individual.
When the host is a prokaryote such as Escherichia coli and the like or a eukaryote such as yeast and the like, the polypeptide of the present invention can be produced, for example, by culturing a transformant harboring a polynucleotide encoding the polypeptide of the present invention in a medium, producing and accumulating the polypeptide of the present invention encoded by the recombinant DNA in a culturing solution, and recovering the polypeptide from the culturing solution.
The method of culturing a transformant harboring a polynucleotide encoding the polypeptide of the present invention in a medium can be performed according to a normal method which is used in culturing of a host.
As a medium in which a transformant obtained by using, as a host, a prokaryote such as Escherichia coli and the like or a eukaryote such as yeast and the like is cultured, any of a natural medium and a synthetic medium may be used as far as it is a medium which contains a carbon source, a nitrogen source, inorganic salts or the like utilizable by the organism and can perform culturing of a transformant effectively.

When a transformant is a prokaryote such as Escherichia coli and the like or a eukaryote such as yeast and the like, as a medium in which the resultant transformant is cultured, any of a natural medium and a synthetic medium may be used as far as it is a medium which contains a carbon source, a nitrogen source, inorganic salts or the like utilizable by a host, and can perform culturing of a transformant effectively. As a medium upon culturing of a transformant for which a host is Escherichia coli, for examples, a YT medium containing bactotryptone, yeast extract and sodium chloride is preferable.

The carbon source may be a carbon source which can be utilized by each microorganism, and carbohydrates such as glucose, fructose, sucrose, molasses containing them, starch and starch hydrolysate and the like, organic acids such as acetic acid propionic acid and the like, and alcohols such as ethanol, propanol and the like can be used.
As the nitrogen source, ammonia, ammonium salts of various inorganic acids and organic acids such as ammonium chloride, ammonium sulfate, ammonium acetate, ammonium phosphate and the like, other nitrogen-containing substances, as well as peptone, meat extract, yeast extract, corn steep liquor, casein hydrolysate, soybean cake and soybean cake hydrolysate, various fermenting bacterial cells and digestion products thereof, and the like can be used.
As the inorganic salt, primary potassium phosphate, secondary potassium phosphate, magnesium phosphate, magnesium sulfate, sodium chloride, ferrous sulfate, manganese sulfate, copper sulfate, calcium carbonate and the like can be used. Culturing is performed under the aerobic condition, such as shaking culture and deep aeration spinner culture.

A culturing temperature is suitably 15 to 40°C, and a culturing time is usually 5 hours to 7 days. During culturing, a pH is retained at 3.0 to 9.0. A pH is adjusted using an inorganic or organic acid, an alkali solution, urea, calcium carbonate, ammonia or the like. If necessary, during culturing, an antibiotic such as ampicillin and tetracycline may be added to a medium.
When a microorganism transformed with an expression vector using an inductive promoter as a promoter is cultured, an inducer may be added to a medium, if necessary. For example, when a transformant transformed with an expression vector using a lac promoter is cultured, isopropyl-β-D-thiogalactopyranoside or the like may be added to a medium and, when a transformant transformed with an expression vector using a trp promoter is cultured, indoleacrylic acid or the like may be added to a medium. A cell or an organ with a gene introduced therein can be cultured at a large scale using a jar fermenter. As a culturing medium, a Murashige and Skoog (MS) medium, a White medium, or a medium obtained by adding a plant hormone such as auxin, cytokinin and the like to these media can be used.

When a transformant for producing the polypeptide of the present invention is an animal cell, as a medium for culturing the cell, a RPMI1640 medium (The Journal of the American Medical Association, 199, 519 (1967)), a MEM medium (Science, 130, 432 (1959)), a D-MEM medium (Virology, 8, 396 (1959)), a 199 medium (Proceedings of the Society for the Biological Medicine, 73, 1 (1959)) or a medium obtained by adding a bovine fetal serum (FCS) or the like to these media, which is usually used, is used.
Culturing is usually performed for 1 to 7 days under the conditions of a pH 6 to 8, 25 to 40°C, the presence of 5% CO₂. In addition, during culturing, an antibiotic such as kanamycin, penicillin, streptomycin and the like may be added to a medium, if necessary.
When a transformant is an insect cell, as a medium for culturing, a TNM-FH medium (manufactured by Pharmingen), a Sf-900II SFM medium (manufactured by Invitrogen), ExCell400, ExCell405 (manufactured by JRH Biosciences), Grace's Insect Medium (Nature, 195, 788 1962)) and the like, which are generally used, can be used.

(vii) Production method
The polypeptide of the present invention can be produced by culturing a transformant, and isolating and purifying the polypeptide of the present invention from a culturing solution. The method of isolating and purifying the polypeptide of the present invention can be performed by a normal method which is well-known and conventional in the art and, for example, a method of isolating and purifying an enzyme, and a method of purifying a glycosyltransferase of Sandler et al. (Methods in Enzymology, 83, 458) can be used.
When the polypeptide of the present invention is produced and accumulated as a soluble polypeptide, a culturing solution in which a transformant has been cultured as described above is separated into a cell or a bacterial cell and a medium by a method such as centrifugation and the like, for example. When the polypeptide of the present invention is present in a host cell, a collected cell or bacterial cell is washed with a suitable buffer such as a STE solution and the like, the cell or the bacterial cell is ground with ultrasound, a French press, Manton Gaurin homogenizer, Dyno mill or the like, and the polypeptide can be obtained as a cell-free solution by centrifugation or filtration.
A buffer used in separation and purification of the polypeptide of the present invention may contain a suitable amount of a surfactant and, for example, may contain sodium laurylsulfate (SDS) and N-lauroylsarcosine sodium (sarcosyl).
A method of separating and purifying an objective protein contained in the resulting crude purified product can be performed by combining the known per se various separation and purification methods. As these known methods, there are exemplified a solvent extraction method, a slating out method with ammonium sulfate or the like, a dialysis method, a precipitation method with an organic solvent, an ultrafiltration method, a gel filtration, various chromatography methods such as dimethylaminoethyl (DEAE)-Sepharose chromatography, anion chromatography and ion-exchange chromatography using lysine such as DIAIONE HPA-75 (manufactured by Mitsubishi Chemical Co., Ltd.) and the like, cation chromatography using lysine such as S-Sepharose FF (manufactured by Pharmacia), hydrophobic chromatography of butyl Sepharose and the like, affinity chromatography and the like, various electrophoreses such as SDS-polyacrylamide gel electrophoreses and isoelectric focusing, and the like.

When the polypeptide of the present invention is produced and accumulated as an insoluble polypeptide, a cell or a bacterial cell is separated, and ground with a suitable method as describe above and a fraction containing the polypeptide is recovered. The recovered sample is solubilized with a solubilizing agent such as a surfactant such as sodium laurylsulfate (SDS) and N-lauroylsarcosine sodium (sarcosyl). The solubilizing solution is diluted or dialyzed to such a concentration that little or no solubilizing agent is contained, and the polypeptide is constructed into a normal steric structure, thereafter, a purified preparation can be obtained by the aforementioned separation and purification method.

Alternatively, the polypeptide of the present invention is produced as a fused protein with other protein, and the polypeptide can be purified utilizing affinity chromatography using a substance having affinity for the fused protein (Akio Yamakawa, Experimental Medicine, 13, 469-474 (1995)). As an addition protein used in a fused protein, there are exemplified Protein A, FLAG and the like (Proceedings of the National Academy of Sciences USA, 86, 8227 (1989), Genes Development, 4, 1288 (1990), JP-A No. 5-336963, JP-A No. 6-823021). When Protein A is used, a fused protein of the polypeptide of the present invention and Protein A is produced, and the polypeptide can be purified by performing affinity chromatography using immunoglobulin G. When the FLAG peptide is used, a fused protein of the polypeptide of the present invention and FLAG is produced, and the polypeptide can be purified by performing affinity chromatography using an anti-FLAG antibody.

The polypeptide of the present invention can be produced using an in vitro transcription/translation system according to the known method (Journal of Biomolecular NMR, 6, 129-134 (1995), Science, 242, 1162-1164 (1988), The Journal of Biochemistry, 110, 166-168 (1991)).
The polypeptide of the present invention can be chemically synthesized, based on its amino acid sequence, by a chemical synthesizing method such as a Fmoc method (fluorenylmethyloxycarbonyl method), a tBoc method (t-butyloxycarbonyl method) and the like, or commercially available peptide synthesizer, such as a peptide synthesizer such as APEX396 (manufactured by Advanced Chemtech), 433A (manufactured by Applied Biosystems), PS3 (manufactured by Protein Technology), 9050 (manufactured by Perceptive), PSSM-8 (manufactured by Shimadzu Corporation) and the like.
Analysis of a structure of the polypeptide of the present invention can be performed by a method which is usually used in protein chemistry, for example, the method described in Protein Structural Analysis for Gene Cloning (Hisashi Hirano, published by Tokyo Kagaku Dozin Co., LTD. in 1993). Chordin activity of the polypeptide of the present invention can be measured according to the known measuring method (The Journal of Biological Chemistry, 258, 9893-9898 (1983), The Journal of Biological Chemistry, 262, 15649-15658 (1987), The Journal of Biological Chemistry , 273, 58-65 (1998), The Journal of Biological Chemistry, 273, 433-440 (1998), The Journal of Biological Chemistry, 273, 12770-12778 (1998), Archives of Biochemistry and Biophysics, 270, 630-646 (1989), Archives of Biochemistry and Biophysics, 274, 14-25 (1989), JP-ANo. 6-181759).

Method for producing mutant polypeptide
Deletion or substitution of an amino acid in the polypeptide of the present invention can be performed by a site-specific mutagenesis method which is the well-known technique before filing of the present application. Deletion or substitution of one or a few of amino acids can be prepared according to the method described in Molecular Cloning: A Laboratory Manual, Second Edition (1989) (Cold Spring Harbor Laboratory Press), Current Protocols in Molecular Biology (1994) (Wiley-Interscience), Nucleic Acids Research, 10, 6487 (1982), Proceedings of the National Academy of Sciences USA, 79, 6409 (1982), Gene, 34, 315 (1985), Nucleic Acids Research, 13, 4331 (1985), Proceedings of the National Academy of Sciences USA, 82, 488 (1985), Proceedings of the National Academy of Sciences USA, 81, 5662 (1984), Science, 224, 1431 (1984), WO 85/00817, Nature, 316, 601 (1985).

### Production of antibody recognizing polypeptide of the present invention

(i) Production of polyclonal antibody
An antibody can be produced by administering, as an antigen, a full length polypeptide of the present invention, a partial peptide thereof or a polypeptide comprising the partial peptide thereof to a mammal. The antigen may be itself, or an antigen bound to a carrier, for example, bovine serum albumin (BSA), keyhole limpet hemocyanin (KLH) or bovine thyroglobulin (BTG) may be used. In addition, in order to enhance an immunological reaction with an antigen, for example, Freund's complete adjuvant (CFA) or incomplete adjuvant (IFA) may be administered. As a mammal used in immunization, mouse, rat, rabbit, gout, hamster and the like can be used.
A polyclonal antibody can be produced according to, for example, the method of Lane et al. (Antibodies: A Laboratory Manual, Second Edition (1989) (Cold Spring Harbor Laboratory Press).
After an antigen is first administered to a mammal, the antigen is administered 3 to 10 times every 1 to 2 weeks to obtain an immunized mammal, and a serum is collected from the mammal, and purified, thereby, the antibody can be produced.
Administration of the antigen is performed 3 to 10 times every 1 to 2 weeks after first administration. A dose of the antigen is preferably 50 to 100 µg per animal/one time. When a peptide is used, it is desirable to use an antigen covalently bound to a suitable carrier. A peptide which is to be an antigen can be synthesized by a genetic engineering procedure or a peptide synthesizer. After each administration, blood is collected from an eyeground venous plexus on Day 3 to 7, and a reaction of the serum with an antigen used in immunization can be confirmed by the method described in enzyme-liked immunosorbent assay (ELISA method: published by Igaku-Shoin Ltd. (1976), Antibodies: A Laboratory Manual, Second Edition (1989) (Cold Spring Harbor Laboratory Press).
Blood is collected from an immunized mammal, and an antibody titer is measured. At a time point at which the mammal is immunized to such an extent that a sufficient antibody titer is obtained, blood is collected, and serum is prepared, thereby, a polyclonal antibody can be obtained. A method of separating and purifying a polyclonal antibody can be performed by centrifugation, salting out with ammonium sulfate, caprylic acid precipitation (Antibodies: A Laboratory Manual, Second Edition (1989) (Cold Spring Harbor Laboratory Press) or various chromatographies using a DEAE-Sepharose column, an anion exchange column, a protein A column, a G-column or a gel filtration column, alone or in combination thereof.

(ii) Production of monoclonal antibody
(a) Preparation of antibody producing cell
   A monoclonal antibody-producing hybridoma can be obtained by obtaining a sufficient antibody titer in (i), thereafter, collecting spleen or lymph node from the mammal, and fusing an antibody-producing cell obtained therefrom with a myeloma cell. As a myeloma cell, a cell strain established from a mouse or a rat is used. A method of cell fusion can be performed by the known method, and a fused cell can be produced according to a Koehler and Milstein method (Nature, 256, 495-497 (1975)).
   The polypeptide of the present invention, a partial peptide thereof or a polypeptide comprising the partial peptide thereof is administered, an antigen substance is finally administered to a rat exhibiting a sufficient antibody titer, thereafter, a spleen as an antibody-producing cell is isolated on Day 3 to 7. The spleen is finely cut in a MEM medium (manufactured by Nissui Pharmaceutical Co., LTD), loosened with a pincette, and centrifuged at 1, 200 rpm for 5 minutes to obtain a precipitation fraction. The spleen cell in the obtained precipitation fraction is treated with a Tris-ammonium chloride buffer (pH 7.65) for 1 to 2 minutes to remove erythrocyte, this is washed with a MEM medium three times, and the resulting spleen cell is used as an antibody-producing cell.

(b) Preparation of myeloma cell
As the myeloma cell, an established cell derived from a mouse or a rat is used and, as such the cell, for example, a 8-azaguanine-registant mouse (BALB/c-derived) myeloma cell, a P3-X63Ag8-U1 strain (hereinafter, abbreviated as P3-U1) (Current Topics Microbiological Immunology, 81, 1 (1978), European Journal of Immunology, 6, 511 (1976)), a SP2/0-Ag14 strain (hereinafter, abbreviated as SP-2) (Nature, 276, 269 (1978)), a P3-X63-Ag8653 strain (hereinafter, abbreviated as 653) (Journal of Immunology, 123, 1548 (1979)), P3-X63-Ag8 (hereinafter, abbreviated as X63) (Nature, 256, 495 (1975)) and the like can be used. These cell strains are subcultured in a 8-azaguanine medium (a normal medium containing 15 µg/ml 8-azaguanine (RPMI1640 medium containing 1.5 mM glutamine, 5 x 10 ⁵M 2-mercaptoethanol, 10 µg/ml gentamycin and 10% FCS (manufactured by CSL)), and is cultured in a normal medium 3 to 4 days before cell fusion. In cell fusion, 2 x 10⁷ or more of the thus prepared cells are used.

(c) Production of hybridoma
The antibody-producing cell prepared in (a) and the myeloma cell prepared in (b) are washed with a MEM medium or PBS (per 1 litter; 1.83g disodium phosphate, 0.21g monopotassium phosphate, 7.65g NaCl, pH7.2), they are mixed at the number of the antibody-producing cell which is 5 to 10-fold the number of myeloma cell, and centrifuged at 1, 200 rpm for 5 minutes to obtain a precipitation fraction. A cell group of the resulting precipitation fraction is loosened well, to the cell group is added 0.2 to 1 ml of a polyethylene glycol solution (2g polyethylene glycol-1000 (peg-1000), 2 ml MEM medium, 0.7 ml dimethyl sulfoxide (DMSO) per 10⁸ of the antibody-producing cell at 37°C while stirring and, further, 1 to 2 ml of a MEM medium is added a few times every 1 to 2 minutes. A total amount is prepared with a MEM medium to 50 ml, and this is centrifuged at 900 rpm for 5 minutes to obtain a precipitation fraction. To the precipitation fraction is added 100 ml of a HAT medium (normal medium, containing 10⁻⁴M hypoxanthine, 1.5 x 10⁻⁵M thymidine and L x 10⁻⁷M aminopterin), and this is slowly loosened, and is suspended.
Each 100µl of the suspension is dispensed in each well of a 96-well plate for culturing, and this is cultured at 37°C for 7 to 14 days in the presence of 5% CO₂, and a hybridoma which produces an antibody specifically reacting the polypeptide of the present invention among an antibody produced in the culturing supernatant is selected by the method described in enzyme-linked immunosorbent assay (Antibodies: A Laboratory Manual, Second Edition (1989) (Cold Spring Harbor Laboratory Press)).

### Method of immunologically detecting polypeptide of the present invention

Examples of the method of immunologically detecting the polypeptide of the present invention include a method of measuring the polypeptide or a partial peptide thereof using a label body in which an antibody to the polypeptide or a partial peptide thereof is directly or indirectly bound to an enzyme a fluorescent substance a radioactive isotope, a latex or the like. As such the measuring method, there are exemplified an ELISA method and a chemiluminescent method of detecting an enzyme label such as horseradish peroxidase and alkaline phosphatase, a FITE method detecting a fluorescent label such as luminol and GFP (Green Fluorescence Protein), a RIA method detecting a radioactive isotope label such as ¹²⁵I, a latex aggregation method using a label bound to a latex.
As such the measuring method, there are also exemplified a Western blotting method and immunohistological staining.
In addition, in such the method of detecting or measuring the polypeptide of the present invention, an aptamer to the peptide or a partial peptide thereof can be used, in place of an antibody to the peptide or a partial peptide thereof.
Further, by using such the measuring method, the polypeptide of the present invention or a partial peptide thereof can be also quantitated.
An antibody used in an immunological detecting method may be immobilized on a solid phase support, and a bound polypeptide may be detected using a secondary antibody or a reagent having a reporter group. Alternatively, the polypeptide of the present invention is labeled with a reporter group, this is reacted with an antibody and a sample, thereafter, the polypeptide can be also detected by a competitive method in which the polypeptide is connected with an immobilized antibody. An extent of inhibition of binding of the same labeled polypeptide with an antibody by the polypeptide of the present invention in a sample is indicated by reactivity of a sample with an immobilized antibody, and a concentration of the polypeptide of the present invention in a sample can be calculated. As the solid phase support, any substance can be used as far as it is an arbitrary substance known to a person skilled in the art, to which an antibody can be attached, and examples include a microtiter plate, a membrane such as a nitrocellulose membrane, beads, a disk, for example, a glass, a glass fiber, a latex, and a plastic substance such as polystyrene and polyvinyl chloride. A magnetic particle or a fiber optical sensor (US 5359681 etc.) may be also used. As a method of immobilizing an antibody on a support, the method known in the art can be used. Herein, the "solid phase" means immobilization by a physical method such as adsorption and the like, or a chemical method with a covalent binding of an antibody with a functional group on a support. An antibody and a functional group on a support may be bound directly or may be bound via a cross-linking agent.

Immobilization by a physical method can be attained by contacting an antibody appropriately diluted in a suitable buffer with a support, preferably, a microtiter plate or a membrane for a suitable time. A contacting time varies depending on a temperature, and is typically between about 1 hour and one day. About 10ng to about 1µg preferably about 100 to 200ng of an antibody is added to each well of a microtiter plate made of a plastic such as polystyrene and polyvinyl chloride, to immobilize an antibody.
Immobilization by a chemical method can be attained by reacting a support and a functional group of an antibody, for example, a bifunctional reagent reacting with both of a hydroxy group and an amino group with a support. For example, an antibody can be solid-phased by covalent binding by using benzoquinone in a support having a suitable polymer coating, or fusing an aldehyde group on a support and amine and active hydrogen on a binding partner. Such the procedure can be performed, for example, with reference to Pierce Immunotechnology Catalogue and Handbook (1991) A12-A13.
A support with which an antibody is solid-phased is treated with a suitable blocking agent, for example, bovine serum albumin, Tween 20 (manufactured by Sigma Aldrich) or the like by the method known to a person skilled in the art, in order to inhibit physical adsorption of other polypeptide.
The antibody-solid phased support and a sample are reacted to bind the polypeptide of the present invention and an antibody. A sample may be appropriately diluted with a suitable diluent, for example, a phosphate-buffered saline (PBS) or the like. A time for a reaction between a sample and an antibody is a sufficient time to detect the presence of the polypeptide of the present invention in a sample derived from an individual with a cancer, preferably, a time reaching at least a 95% binding level of a binding level attaining equilibrium between a bound polypeptide and an unbound polypeptide. A time attaining equilibrium can be easily determined by measuring a binding level with time. Substances other than the bound polypeptide can be removed by washing a solid phase support with a suitable buffer, for example, PBS (containing 0.1% Tween 20) or the like. A labeled secondary antibody and a solid phase support are reacted. As the label, preferably, an enzyme such as horseradish peroxidase and the like, a substrate, a cofactor, an inhibitor, a dye, a radioactive isotope, a coloring substance, a fluorescent substance and the like are used. An antibody and a label are bound by the method known to a person skilled in the art. A secondary antibody is reacted for a sufficient time for binding to a complex of a solid phase antibody and the polypeptide of the present invention. A suitable time can be easily determined by measuring a binding label with time. An unbound secondary antibody can be removed by washing a solid phase support with a suitable buffer, for example, PBS (containing 0.1%, Tween 20) or the like. A method of detecting a label of a secondary antibody is different depending on a kind of a label. When a radioactive isotope is used as a label, detection with a scintillation counter or autoradiography is used. When a dye, a coloring substance or a fluorescent substance is used as a label, detection with a spectrophotometer is used. When an enzyme is used as a label, a substrate to it is added, and a product after a reaction for a certain time is detected with a spectrophotometer or the like. A label and a secondary antibody may be directly bound, or a label and a secondary antibody which are indirectly bound with avidin-biotin or the like may be used. In this case, detection becomes possible by binding one of them to a secondary antibody, and labeling the other.

In addition, the polypeptide of the present invention can be detected by a flow-through test or a strip test. An antibody is immobilized on a membrane such as a nitrocellulose membrane and the like and, in the flow-through test, a sample is added thereto and, when a sample has passed through a membrane, the polypeptide is bound to a solid phase antibody to form an immunological complex and, when a solution containing a labeled secondary antibody has passed through a membrane, it is bound to the immunological complex. In the strip test, a sample is added and, when a sample has passed through a region containing a labeled antibody, the polypeptide is bound with the labeled antibody to form an immunological complex and, when the sample has passed through a region containing a solid phase antibody, the immunological complex is bound, and an amount of a secondary antibody detected in a solid phase antibody region indicates the presence or the absence of a cancer. A label at a detection site can be visually confirmed and, when a label can not be confirmed, negative is indicated. An amount of an antibody which is solid phased with a membrane is preferably about 25 ng to about 1 µg, more preferably about 50 ng to about 1 µg.

### The method of detecting disease

The polypeptide of the present invention, since expression is increased in an angiogenesis model, can be utilized in diagnosing diseases associated with angiogenesis, for example, a vascular disease, an inflammatory disease, an entoptic neovascular disease, a reproductive disease, a central nervous system disease or a cancer. Further, it was found out that expression of the polypeptide is increased in an ovary cancer and a large intestine cancer tissue, and the polypeptide can be utilized in diagnosing these cancers. The presence or the absence of a cancer of a patient can be determined by specifically detecting the polypeptide of the present invention from a biological sample, for example, a cancer or a normal tissue biopsy, an excised tissue, blood, a lymph node, serum, urine or other tissue obtained from a patient, a homogenate or an extract from them or the like, and comparing its expression amount with an expression amount of a healthy person or a normal tissue.
Examples of a method of detecting a disease include a method of immunologically detecting the polypeptide.
Examples of the method of immunologically detecting the polypeptide include a sandwich ELISA method in which one of two kinds of antibodies recognizing different epitopes reacting the polypeptide is directly or indirectly labeled with an enzyme, and this is used, a competitive RIA method in which the polypeptide labeled with a radioactive isotope such as ¹²⁵I and the like, and an antibody recognizing it are used, and the like.

The presence or the absence of a cancer can be attained by measuring and comparing the polypeptide of the present invention detected in a normal person sample and a patient sample. Generally, letting an average of the polypeptide of the present invention obtained from samples derived from patients not having the known established cancer from a non-cancerous tissue to be a cutoff value, a detected value of the polypeptide of the present invention from a sample and a cutoff value are compared. Generally, when a detected value exceeds a value obtained by adding a 3-fold standard deviation value to a cutoff value, the sample is considered positive for a cancer. In addition, a cutoff value is determined using Receiver Operator Curve in accordance with the method of Sackett et al., Clinical Epidemiology: A Basic Science for Clinical Medicine 106-107, (1985) (Little Brown and Co).

### Diagnostic kit

The diagnostic kit of the present invention contains the polypeptide of the present invention as a standard reagent. Diseases associated with angiogenesis, for example, a vascular disease, an inflammatory disease, an entoptic neovascular disease, a reproductive disease, a central nervous system disease or cancer such as an ovary cancer and a large intestine cancer can be diagnosed also by an immunological procedure using an antibody to the polypeptide of the present invention.

### Method of screening binding substance

The polypeptide of the present invention is useful as a reagent for searching or screening a binding substance thereto. That is, a method of screening a binding substance to the polypeptide of the present invention comprising contacting the polypeptide with a test substance is provided. As a test substance, for example, a natural or chemical synthesized chemical substance, a protein, a tissue extract of a mammal such as a human, a mouse, a rat, a pig, a horse, a sheep, a monkey and the like, a cell culture and the like are used. These test substances together with the polypeptide of the present invention are added, fractionation is performed while the DNA synthesis activity of the vascular endothelial cell is measured, a single ligand can be finally obtained.
Specifically, as the method of screening a binding substance of the present invention, there is exemplified a method of using the polypeptide of the present invention or a partial peptide thereof, or constructing an expression system of a recombinant polypeptide, and using a binding assay system using the expression system, or measuring the cell stimulation activity by binding with the polypeptide of the present invention, for example, the activity of promoting or inhibiting DNA synthesis due to uptake of BrdU (bromodeoxyuridine), the cell wandering activity or the like, or visually measuring lumen formation of a vascular endothelial cell with a microscope or the like.
First, as the polypeptide used in the method of screening a binding substance, any polypeptide can be used as far as it contains the polypeptide of the present invention, and a polypeptide obtained by expressing at a large amount using an animal cell is suitable. For producing the polypeptide of the present invention, the aforementioned expression method is used, and it is preferable to perform the method by expressing a DNA encoding the polypeptide in a mammal cell or an insect cell. As a DNA fragment encoding an objective polypeptide part, a complementary DNA is usually used, being not limiting. For example, a gene fragment and a synthetic DNA may be used. For introducing a DNA fragment encoding the polypeptide of the present invention into a host animal cell, and effectively expressing it, it is preferable to incorporate the DNA fragment downstream of a polyhedron promoter of nuclear polyhedrosis virus (NPV) belonging to a vaculovirus for which an insect is host, a SV40-derived promoter, a promoter of a retrovirus, a metallothionein promoter, a human heat shock promoter, a cytomegalovirus promoter, a SRα promoter or the like. An amount and quality of the expressed polypeptide can be tested by the known per se method. For example, it can be performed according to the method described in The Journal of Biological Chemistry, 267, 19555-19559 (1992).
Therefore, in the method of screening a binding substance of the present invention, as an entity containing the polypeptide of the present invention or a partial peptide thereof, the polypeptide purified according to the known per se method or a partial peptide thereof may be used or a cell containing the polypeptide or a supernatant of the cell may be used. When a cell containing the polypeptide of the present invention is used in the method of screening a binding substance of the present invention, the cell may be immobilized on an agarose gel or the like. An immobilization method can be performed according to the known per se method. The cell containing the polypeptide of the present invention refers to a host cell expressing the polypeptide of the present invention and, as the host cell, Escherichia coli, Bacillus subtilis, yeast, an insect cell, an animal cell and the like are used.
For screening a binding substance to the polypeptide of the present invention, a suitable polypeptide fraction, and a labeled test substance are necessary. As the polypeptide fraction, a natural polypeptide fraction, or a recombinant polypeptide fraction having the activity equivalent thereto is desirable. Herein, the equivalent activity indicates the equivalent ligand binding activity, signal information transmitting action or the like. Examples of the labeled test substance include test substances labeled with ³H, ¹²⁵I, ¹⁴C, ³⁵S or the like.
Specifically, for performing the method of screening a binding substance to the polypeptide of the present invention, first, a cell or a cell membrane fraction containing the polypeptide of the present invention is suspended in a buffer suitable for the screening method to prepare a polypeptide preparation. As the buffer, any buffer can be used as far as it is a buffer which does not inhibit binding of the ligand and the polypeptide, such as a phosphate buffer, Tris-HCl buffer and the like having a pH of 4 to 10 preferably a pH of 6 to 8. In addition, in order to reduce non-specific binding, a surfactant such as CHAPS, Tween-80 (Kao-Atlas), digitonin, deoxycholate and the like, and various proteins such as bovine serum albumin and gelatin may be added to the buffer. Further, in order to suppress degradation of a receptor or a ligand with a protease, a protease inhibitor such as PMSF, leupeptin, E-64 (manufactured by Peptide Institute, Inc.), pepstatin and the like may be added. A test substance labeled with a constant amount, preferably 5000 cpm to 500000 cpm of ³H, ¹²⁵I, ¹⁴C, ³⁵S or the like is present in 0.01 to 10 ml of the polypeptide solution. In order to know a non-specific binding amount (NSB), a reaction tube to which a considerable excess of an unlabeled test substance has been added is also prepared. A reaction is performed at 0°C to 50°C, preferably 4°C to 37°C for 20 minutes to 24 hours, preferably 30 minutes to 3 hours. After the reaction, the sample is filtered with a glass fiber filter or the like, and washed with a suitable amount of the same buffer, and radioactivity remaining on the glass fiber filter is measured with a liquid scintillation counter or a γ-counter. A test substance having a count (B-NSB) obtained by subtracting a non-specific binding amount (NSB) from a total binding amount (B), exceeding 0 cpm, can be selected as a binding substance to the marker of the present invention.
In order to screen a binding substance to the polypeptide of the present invention, the cell stimulation activity through the polypeptide, for example, the activity of promoting or suppressing DNA synthesis due to uptake of BrdU (bromodeoxyuridine), the cell wandering activity or the like can be measured using the known method or a commercially available measuring kit. Alternatively, lumen formation of a vascular endothelial cell can be also measured by a method of visual measurement with a microscope. Specifically, first, a cell containing the polypeptide is cultured in a multiwell plate or the like. Upon screening of a binding substance, a medium and a buffer are exchanged in advance with a fresh medium or a suitable buffer exhibiting no toxicity to a cell, respectively, a test substance or the like is added, and this is incubated for a constant time, thereafter, a cell is extracted or a supernatant is recovered and the generated product is quantitated according to each method.

### Method of screening binding activity regulating substances

The polypeptide of the present invention is useful as a reagent for searching or screening a binding activity regulating substance to the polypeptide. A method of screening a substance which regulates the activity of binding between the polypeptide and a binding substance, that is, a substance which changes the binding property, a recombinant expression system expressing the polypeptide of the present invention is constructed and, using a bioassay system or a binding assay system, a substance which changes the binding property between a binding substance and the polypeptide of the present invention, for example, a peptide, a protein, a non-peptidic substance, a synthetic substance, a fermentation product and the like can be effectively screened. Examples of such the substance include (a) a substance which enhances or decreases the cell stimulation activity via a binding substance and a polypeptide of the present invention, for example, the activity of promoting or suppressing DNA synthesis due to BrdU (bromodeoxyuridine), the activity of promoting or suppressing the cell wandering activity or the like, and (b) a substance which enhances or decreases a binding force between a binding substance and the polypeptide of the present invention.
That is, the present invention provides a method of screening a substance which regulates the activity of binding between a binding substance and the polypeptide of the present invention, comprising comparing a binding amount of a binding substance, between (i) the case where the marker of the present invention and a binding substance are contacted, and (ii) the case where the polypeptide of the present invention, a binding substance and a test substance are contacted.
The method of screening a binding activity regulating substance of the present invention comprises comparing an amount of binding of the polypeptide and a binding substance by, for example, measuring the cell stimulation activity or the like, in cases of (i) and (ii).
Examples of the method of screening a binding activity regulating substance of the present invention include (a) a method of screening a binding activity regulating substance which changes the binding property between a binding substance and the polypeptide of the present invention, comprising measuring and comparing an amount of binding of a labeled binding substance to the polypeptide, in the case where a labeled binding substance is contacted with the polypeptide of the present invention, and the case where a labeled binding substance and a test substance are contacted, (b) a method of screening a binding activity regulating substance which changes the binding property between a binding substance and the polypeptide of the present invention, measuring and comparing an amount of binding of a labeled binding substance to the polypeptide in a cell or a cell culturing solution containing the polypeptide of the present invention, in the case where the cell or the cell culture solution is contacted with a labeled binding substance, and the case where a labeled binding substance and a test substance are contacted, (c) a method of screening a binding activity regulating substance which changes the binding property between a binding substance and the polypeptide of the present invention, comprising measuring and comparing an amount of binding of a labeled binding substance to the polypeptide, in the case where a polypeptide secreted into a cell culture by culturing a transformant containing a polynucleotide encoding the polypeptide of the present invention, and a labeled binding substance are contacted, and the case where a labeled binding substance and a test substance are contacted, and the like.

### Method of screening expression regulating substance

The polypeptide of the present invention is useful as a reagent for searching or screening an expression regulating substance to the polypeptide of the present invention.
That is, the expression regulating substance screening method of the present invention can screen an expression regulating substance of the marker of the present invention, for example, by measuring a mRNA amount or a protein amount of the polypeptide of the present invention or a partial peptide thereof contained in (i) blood, a particular organ, or a tissue or a cell isolated from an organ of a non-human mammal, or (ii) a transformant.

Specifically, (i) a drug, for example, an anti-cancer agent or the like is administered to a normal or disease model non-human mammal, for example, a mouse, a rat, a rabbit, a sheep, a pig, a cow, a cat, a dog, a monkey or the like and, after a constant time has passed, blood, a particular organ, for example, brain, kidney, large intestine, ovary or the like, or a tissue or a cell isolated from an organ is obtained. A mRNA of the polypeptide of the present invention or a partial peptide thereof contained in the resulting cell is extracted, for example, by the extraction method well-known in the art, and can be quantitated by using, for example, the procedure of TaqManPCR or the like, or can be also analyzed by the well-known Northern blotting method, or (ii) a transformant expressing the polypeptide of the present invention or a partial peptide thereof is prepared according to the aforementioned method, and a mRNA of the polypeptide of the present invention or a partial peptide thereof contained in the transformant can be quantitated and analyzed similarly.

The present invention also includes a "screening kit" which can be used in the aforementioned screening method. The screening kit contains at least the polypeptide of the present invention, the gene of the present invention, a nucleic acid construct harboring the gene, a cell in which the gene is introduced, an antibody or an aptamer to the polypeptide of the present invention, and the like.

Examples of the screening kit of the present invention include:
- a kit containing the polypeptide of the present invention,
- a kit containing a nucleic acid construct comprising the gene of the present invention,
- a kit containing a cell in which the gene of the present invention is introduced,
- a kit containing an antibody to the polypeptide of the present invention or a fragment thereof,
- a kit containing an aptamer to the polypeptide of the present invention, and the like.

In addition, each kit optionally contains the probe and/or a primer pair which can be suitably used in the screening method of the present invention. Further, if desired, a detection reagent, a buffer, a standard substance, an instruction for performing the screening method of the present invention, and the like may be contained.

### Pharmaceutical composition

A pharmaceutical composition (anti-angiogenic agent or angiogenesis inducing agent) containing at least one selected from the polypeptide of the present invention, a neutralizing antibody to the polypeptide, an aptamer of the polypeptide, a compound obtained by using the aforementioned screening method or screening kit can be administered alone as a preventing or treating agent, but usually, it is desirable to provide as a pharmaceutical preparation produced by mixing with pharmacologically acceptable one or more carriers, and formulating into a preparation by an arbitrary method well-known in the technical field of pharmacy. The pharmaceutical composition of the present invention exerts the treating or preventing effect on diseases associated with angiogenesis through expression of the polypeptide of the present invention itself (having anti-angiogenic activity) or the action on expression of the polypeptide (e.g. promoting or suppressive exertion on the expression). Since the polypeptide of the present invention inhibits DNA synthesis of a vascular endothelial cell, and inhibits the cell wandering activity, a DNA synthesis inhibiting agent and a cell wandering activity inhibiting agent are also included. Although the pharmaceutical composition containing the polypeptide of the present invention can be administered alone as a treating drug, it is usually desirable to provide a pharmaceutical preparation produced by mixing with pharmacologically acceptable one or more carriers, and formulating into a preparation by an arbitrary method well-known in the technical field of pharmacy.

The pharmaceutical composition of the present invention may further contain various adjuvants which can stably retain the polypeptide, a neutralizing antibody, an aptamer or a compound. Specifically, examples include an adjuvant, an excipient, a binder, a stabilizer, a buffer, a solubilizer, an isotonic and the like, which exhibit a nature of a suppressing degradation of an active ingredient until an active ingredient reaches a delivery subject site, and are pharmaceutically acceptable.

As an additive which can be kneaded into tablets, capsules or the like, for example, a binder such as gelatin, corn starch, tragacanth, and gum arabic, an excipient such as crystalline cellulose, a bulking agent such as corn starch, gelatin, alginic acid and the like, a lubricant such as magnesium stearate, a sweetener such as sucrose, lactose and saccharine, a flavor agent such as peppermint, an oil of Gaultheria adenothrix and a cherry are used. When a formulation unit form is a capsule, in addition to the aforementioned type of materials, a liquid carrier such as an oil or a fat may be further contained. A sterile composition for injection can be formulated according to a conventional formulation practice such as dissolution or suspension of an active substance in a vehicle such as water for injection, a naturally occurring vegetable oil such as a sesame oil, a coconuts oil and the like. As an aqueous solution for injection, for example, an isotonic of D-sorbitol, D-mannitol, sodium chloride and the like, containing a saline, glucose and an adjuvant are used, and a suitable solubilizer, for example, alcohols such as ethanol, propylene glycol and polyethylene glycol, a nonionic surfactant such as Polysorbate 80 and HCO-50 and the like may be used together. As an oily solution, for example, a sesame oil, a soybean oil and the like are used, and benzyl benzoate, benzyl alcohol and the like which are a solubilizer may be used together.

In addition, the pharmaceutical composition may be blended with, for example, a buffer such as a phosphate buffer, a sodium acetate buffer and the like, a soothing agent such as benzalkonium chloride, procaine hydrochloride and the like, a stabilizer such as human serum albumin, polyethylene glycol and the like, a preservative such as benzyl alcohol, phenol and the like, an antioxidant and the like. The prepared injectable is usually filled into a suitable ample. Since the thus obtained preparation is safe and low in toxicity, it can be administered to a mammal such as a human, a rat, a mouse, a rabbit, a sheep, a pig, a cow, a cat, a dog, a monkey and the like.

It is desirable to use an administration route which is most effective upon treatment, and examples include oral administration, and parenteral administration such as intraoral, intraairway, rectal, subcutaneous, intramuscular and intravenous administration. Examples of a dosage form include sprays, capsules, tablets, granules, syrups, emulsions, suppositories, injectables, ointments, tape agents and the like.

Examples of a preparation which is suitable for oral administration include emulsions, syrups, capsules, tablets, powders, granules and the like. For example, a liquid preparation such as emulsions and syrups can be produced using, as an additive, water, sugars such as sucrose, sorbitol, fructose and the like, glycols such as polyethylene glycol, propylene glycol and the like, oils such as a sesame oil, an olive oil, a soybean oil and the like, antiseptics such as p-hydroxybenzoic acid esters and the like, flavors such as strawberry flavor, peppermint and the like. Capsules, tablets, powders, granules and the like can be produced using, as an additive, excipients such as lactose, glucose, sucrose, mannitol and the like, disintegrating agents such as starch, sodium alginate and the like, lubricants such as magnesium stearate, talc and the like, binders such as polyvinyl alcohols, hydroxypropylcellulose, gelatin and the like, surfactants such as fatty acid ester and the like, plasticizers such as glycerin and the like.

Examples of a preparation suitable for parenteral administration include injectables, suppositories, sprays and the like. For examples, injectables are prepared using a carrier consisting of a salt solution, a glucose solution, or a mixture of both of them. Suppositories are prepared using a carrier such as cacao butter, hydrogenated fat and carboxylic acid. In addition, sprays are prepared using the substance itself, or a carrier which does not stimulate an oral cavity and an airway mucosa of a recipient, and disperses the substance as fine particles to facilitate absorption. Examples of the carrier include lactose, glycerin and the like. Due to a nature of the substance and a carrier used, it is possible to prepare a preparation such as aerosols, dry powders and the like. In addition, also in these parenteral agents, components exemplified as an additive for oral agents can be also added.

Pharmacological assessment of the pharmaceutical composition of the present invention can be performed, for example, by a method of administering the pharmaceutical composition of the present invention to an angiogenesis-associated disease model mouse, and assessing using, as an index, the case where improvement in an angiogenesis-associated disease is seen in an administered animal as compared with a not-administered animal.

Alternatively, the pharmaceutical composition of the present invention may be used by adding to a food or a feed. The "food" and the "feed" refer to a natural product containing one or more kinds of nutrients and a processed product thereof, and include all drinks and foods. A food and a feed with the pharmaceutical composition of the present invention incorporated therein is useful as a health assisting functional food for preventing and/or treating diseases associated with angiogenesis.

An expression vector for suppressing angiogenesis or an expression vector inducing angiogenesis, containing at least one selected from the gene of the present invention and a compound (nucleic acid or derivative thereof) obtained using the screening method or the screening kit exerts the treating or preventing effect on angiogenesis-associated diseases.

A method of producing an expression vector of the present invention, a method of expression in a cell and the like are the same as those of the expression vector described in the aforementioned "method of obtaining the gene of the present invention" and "method of producing the polypeptide of the present invention".

Since the expression vector of the present invention is safe and low in toxicity, it can be administered to, for example, a mammal (e.g. human, rat, mouse, rabbit, sheep, pig, cow, cat, dog, monkey etc.). It is preferable to use a DNA or RNA virus vector or a plasmid vector which can express a protein in a cell of a mammal including a human, and is highly safe, when used in gene therapy. Examples of an applicable virus vector in gene therapy include adenovirus, adeno-associated virus (AAV), retrovirus, poxvirus, herpesvirus, herpes simplex virus, lentivirus (HIV), Sendaivirus, Epstein-Barr virus (EBV), vacciniavirus, poliovirus, sindbisvirus, SV40 and the like. Examples of a preferable plasmid in gene therapy include pCAGGS [Gene, 108, 193-200 (1991)], pPK-CMV, pcDNA3.1, pZeoSV (Invitrogen or Stratagene) and the like.

As a method of introducing the expression vector of the present invention into a patient, there are an in vivo method of directly introducing the vector into a body, and an ex vivo method of taking out a certain kind of a cell from a human, introducing a DNA into the cell in vitro, and returning the cell into a body [Nikkei Science, April, 20-45(1994), Gekkan-Yakuji, 36, 23-48(1994), Jikken-igaku, special edition, 12, 15(1994)]. In the present invention, the in vivo method is preferable.

When administered by the in vivo method, the vector is administered by a suitable administration route depending on a disease to be treated, a target organ or the like. For example, the vector can be directly locally administered to a tissue in which a lesion is recognized, or it can be also administered intravenously, intraarterially, subcutaneously, intravascularly, intraperitoneally, endoscopically, or by an aerosol. As an administration method, intravenous or peritoneal administration is preferable. Alternatively, direct injection into a tissue where a lesion is seen is also preferable. A tissue in which a lesion is seen is photographed using an arbitrary means which can be utilized in the art such as nuclear magnetic resonance imaging and computer tomography, and the vector of the present invention can be administered by localization injection, for example.

In addition, by adding a signal sequence to a DNA encoding the polypeptide of the present invention, a secretion protein is formed, therefore, local administration is not necessarily required, and a protein which is produced in a cell and secreted, acts on a remote target organ to generate the anti-angiogenic activity. Therefore, administration to a normal tissue other than a pathological tissue, or a normal cell is also possible. When administered to a human, intravenous administration or intramuscular administration is preferable.

As a form of a preparation of the vector of the present invention (gene therapeutic agent), various preparation forms in conformity with the aforementioned each administration form can be taken. For example, in the case of an injectable containing the DNA of the present invention as an active ingredient, the injectable can be prepared by a conventional method. A base used in a gene therapeutic agent is not particularly limited as far as it is a base which is usually used in an injectable, and examples include distilled water, a salt solution of sodium chloride, or a mixture of sodium chloride and an inorganic salt, a solution of mannitol, lactose, dextran, glucose or the like, a solution of an amino acid such as glycine, arginine and the like, an organic acid solution, and a mixed solution of a salt solution and a glucose solution. Alternatively, an injectable may be prepared as solutions, suspensions or dispersions using an adjuvant such as an osmotic pressure adjusting agent, a pH adjusting agent, a vegetable oil such as a sesame oil, a soy bean oil and the like, lecithin, or a surfactant such as a nonionic surfactant and the like in these bases according to a conventional method. These injectables may be formulated into preparations for dissolution upon use by an operation such as conversion into powders, lyophilization or the like.

A content of a DNA in a preparation is different depending on a disease to be treated, an administration site, an administration time, a desired treating term, an age, and a weight of a patient, and the like and is adjusted appropriately, and in a normal patient (weight 60kg), a content is generally about 0.01 to 2000 mg, preferably 0.1 to 100 mg in terms of a weight of a DNA encoding the polypeptide of the present invention.

### Treating method

Since the aforementioned pharmaceutical composition can be an agent for treating a vascular disease, an inflammatory disease, an entoptic angiogenic disease, a reproductive disease, a central nervous system disease or cancer, a method of treating a disease such as a vascular disease, an inflammatory disease, an entoptic angiogenic disease, a reproductive disease, a central nervous system disease and cancer by administering the pharmaceutical composition by a convenient and appropriate method is provided. As such the method, it is desirable to use a method of most effectively administering the pharmaceutical composition upon treatment, and examples include oral administration, and parenteral administration such as intraoral, intraairway, rectal, subcutaneous, intramuscular and intravenous administration.

Since the treating agent prepared as described above is safe and low in toxicity, it can be administered to a mammal such as a human, a rat, a mouse, a rabbit, a sheep, a pig, a cow, a cat, a dog, a monkey and the like. A dose of the pharmaceutical composition of the present invention is appropriately selected depending on a kind of an active ingredient; an individual, an organ, a local site, and a tissue to be administered; an age, and a weight of an individual to be administered, and the like. An administration method is not particularly limited, but when an active ingredient is a low-molecular compound or a high-molecular compound, an amount of the active ingredient is for example 0.0001 to 1000 mg/kg weight, preferably 0.001 to 100 mg/kg weight; when the active ingredient is a polypeptide or a derivative thereof, the amount is for example 0.0001 to 1000 mg/kg weight, preferably 0.001 to 100 mg/kg weight; when the active ingredient is a nucleic acid or a derivative thereof, the amount is for example 0.00001 to 100 mg/kg weight, preferably 0.0001 to 10 mg/kg weight. And the agent is administered once or plural times per day so that the above amount becomes a one time dose. An administration term is not particularly limited.

The following Examples are provided for illustration, without any limitation. As a genetic engineering method, the method described in Molecular Cloning: A Laboratory Manual, 2nd Edition (Cold Spring Harbor Laboratory) is used if there is not a special provision.

### Example 1

### Analysis of processing site of Vasohibin protein by amino acid substitution

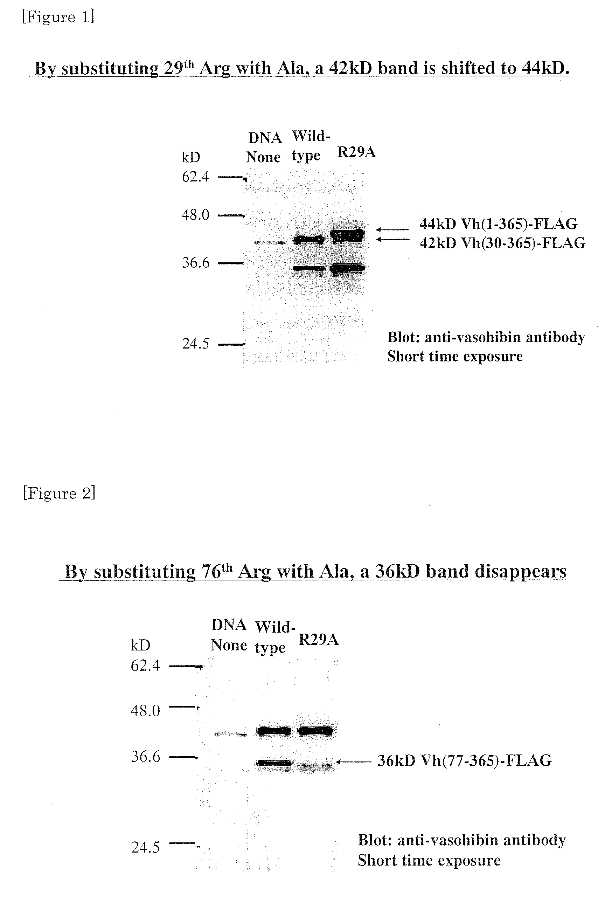

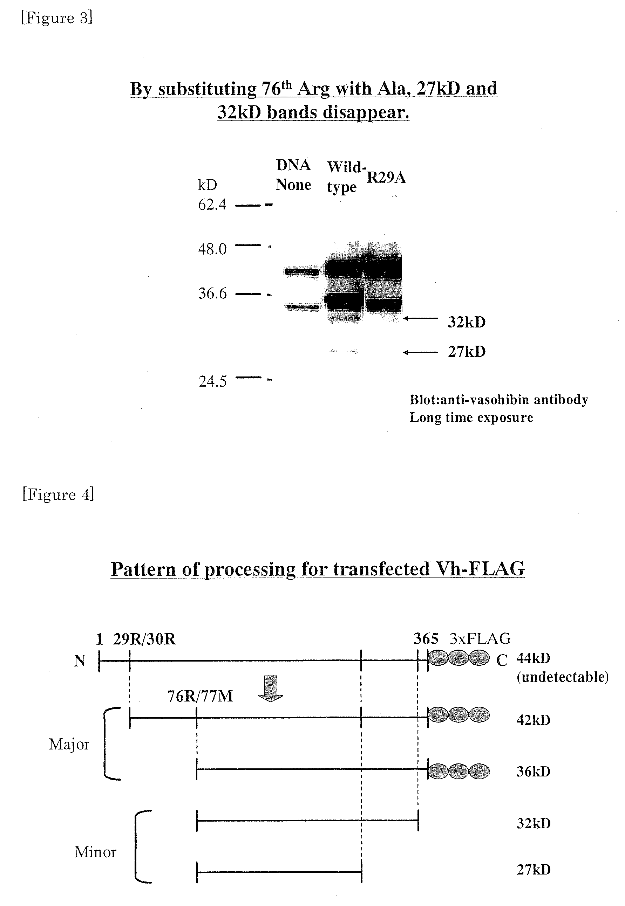

A Vasohibin cDNA was excised in a form connecting to a 3 × FLAG tag from a plasmid pFLAG 14-KIAA1036 described in J.Clin.Invest. 114:898-907, (2004), and inserted into a retrovirus vector pMX to obtain pMX1036. Employing the plasmid pMX1036 as a template, arginine at 29-, 76-, 94-, 96-, 130-, 148-, 296-, 299-, 302-, 318-, 334-, 335-, 338-, 341-, and 342-positions, and lysine at 79-, 83-, 90- and 103-positions among amino acids of SEQ ID No.:2 encoded by the Vasohibin cDNA were substituted with alanine, respectively, with the QuikChange Site-Directed Mutagenesis Kit (Stratagene) to prepare a total of 19 kinds of amino acid-substituted Vasohibin-expressing retrovirus vectors. Each 2 µg of these plasmids together with 0.2 µg of a packaging vector pCLampho (IMGENEX) was introduced into 2.5 × 10⁵ HEK293 cells with a FuGENE6 reagent (Roche Diagnostics), and this was cultured in Dulbecco's modified Eagle medium (Sigma) containing 10% bovine fetal serum for 48 hours. The culturing solution was recovered, diluted 2-fold with the same medium, 10 µg polybrene was added to the resulting solution, and the HUVEC (human umbilical vein endothelial)-derived vascular endothelial cell strain was infected for 48 hours. The same cell was suspended in a RIPA buffer (10 mM Tris-HCl pH 7.5, 150 mM NaCl, 1% deoxycholate, 0.1% SDS, 0.1% Triton X100), and centrifuged at 14500 × g for 10 minutes to obtain the supernatant as a cell extract. For Western blotting, 10 µg of a cell extract was separated with SDS-PAGE according to the method of Laemli, electrically transferred to a nitrocellulose membrane (Tefco), this was blocked in TBS (Tris-HCl containing 0.15 M NaCl, pH7.4) containing a 2% blocking agent (Amersham BioSciences) and 0.05%Triton X-100, and reacted with anti-Vasohibin mAb labeled with HRP. The reaction was washed with TBS containing 0.05% Triton X-100 for 15 minutes four times, chemically light-emitted with an ECL-Advanced reagent (Amersham BioSciences), and this was sensitized on a HyperfilmECL film (Amersham BioSciences) to detect the antibody. Change in a molecular weight of a Vasohibin protein in an amino acid-substituted entity was studied by comparing with that of a wide-type Vasohibin protein with no mutation added. As a result, when 29-positional arginine of SEQ-ID No.:2 was substituted with alanine, a 42 kD band detected in the case of a wild-type Vasohibin protein was shifted to 44 kD (Fig.1). Therefore, it was suggested that a wide-type Vasohibin protein expressed in a cell underwent cutting between a 29-position and a 30-position. In addition, when 76-positional arginine of SEQ-ID No.:2 was substituted with alanine, a 36 kD band detected in the case of the wild-type Vasohibin protein disappeared (Fig.2). Therefore, it was suggested that wild-type Vasohibin protein underwent cutting between a 76-position and a 77-position of SEQ ID No.:2. In addition, 32 kD and 27 kD bands slightly detected in the case of a wild-type became undetected when arginine at 76-position of SEQ ID No.:2 was substituted with alanine, and it was shown that these fragments were generated by cutting between a 76-position and a 77-position of SEQ ID No.:2 (Fig.3). From the above findings, it is thought that processing of a Vasohibin protein in an animal cell is performed in a pattern of Fig.4.

### Example 2

### Expression of Vasohibin (77-365) and Vasohibin (77-318) using baculovirus vector

cDNA encoding Vasohibin (77-365) (region consisting of 77-position to 365-position amino acids of SEQ ID No.:2) and a cDNA encoding Vasohibin (77-318)(region consisting of 77-position to 318-position amino acids of SEQ ID No.:2) were inserted into pFASTBac1(Invitrogen) which is a vector for expressing in an insect cell, to construct insect cell-expressing plasmids pFASTBac-Vh(77-365) and pFASTBac-Vh(77-318). In expression in an insect cell, Bac-To-Bac Baculovirus Expression Systems (Invitrogen) was used, and manipulation was according to the attached manual. That is, constructed pFASTBac-Vh(77-318) and pFASTBac-Vh(77-365) plasmids were transformed into DH10Bac E. coli to obtain a recombinant Bacmid DNA. Then, this Bacmid DNA was transduced into a Sf9 cell with a CELLFECTIN reagent (Invitrogen) to obtain a recombinant baculovirus. A solution (0.5 ml) of this virus was used at such a ratio that 50 ml of a Sf9 cell was infected at a concentration of 1.5 × 10⁶/ml. After infection, the cell was cultured for 76 hours, and the cell was recovered by centrifugation. Confirmation of expression of the Vasohibin (77-318) protein and the Vasohibin (77-365) protein in an insect cell was performed by Western blotting utilizing an extract of this Sf9 cell, and an anti-Vasohibin monoclonal antibody (mAb, 4E12) labeled with HRP. First, a cell was washed with 5 mL of a phosphate buffered saline (PBS) once, suspended in 5 ml of a Lysis solution (50 mM Tris-HCl pH7.4 containing 0.15 M NaCl 0.1 mM EDTA, 0.1 mM EGTA, 1 mM DTT, 0.1 mM amidinophenyl methanesulfonyl fluoride hydrochloride, and 0.1% NP-40), the suspension was ultrasound-treated with MICROCON (manufactured by HEART SYSTEMS) four times for 15 seconds while ice-cooled, centrifuged at 14,500xg minutes for 20 minutes, and an equivalent amount of a Lysis solution was added to the supernatant, which was used as a Lysate solution. For Western blotting, 0.5 µl was separated with SDS-PAGE according to the method of Laemli, electrically transferred to a nitrocellulose membrane (Tefco), blocked in TBS containing a 5% skimmed milk, and reacted with anti-Vasohibin mAb labeled with HRP. The reaction was washed with TBS (Tris-HCl, pH7.4 containing 0.15 M NaCl) containing 0.05% Triton X-100 three times for 10 minutes, this was chemically light-emitted with an ECL-Plus reagent (Amersham BioSciences), and this was sensitized on a HyperfilmECL film (Amersham BioSciences) to detect the antibody. For purifying a protein, an anti-Vasohibin antibody solid phase gel was prepared. The method used a kit of AminoLink Immobilization Kit (PIERCE), and manipulation was according to the attached manual. Purification of a protein was performed by adding a Lysate solution to an anti-Vasohibin antibody column equilibrated with TBS containing 0.05% Tween 20, thereafter, washing this with the same solution, eluting the column with an eluate (6 mM HCl pH2.2 containing 0.02% Tween 20) (0.5 ml/fraction), and immediately neutralizing this with 1 M Tris-HCl, pH8.0 (7 µl, relative to 0.5 ml of an eluate). By Western blotting of each fraction, or by staining with Bio-Safe Coomassie (Bio-Rad) after SDS-PAGE, a single band was confirmed at about 27 kDa and about 33 kDa, respectively, for the Vh(77-318) protein and the Vh(77-365) protein.

### Example 3

### In vitro anti-angiogenic effect with protein of the present invention

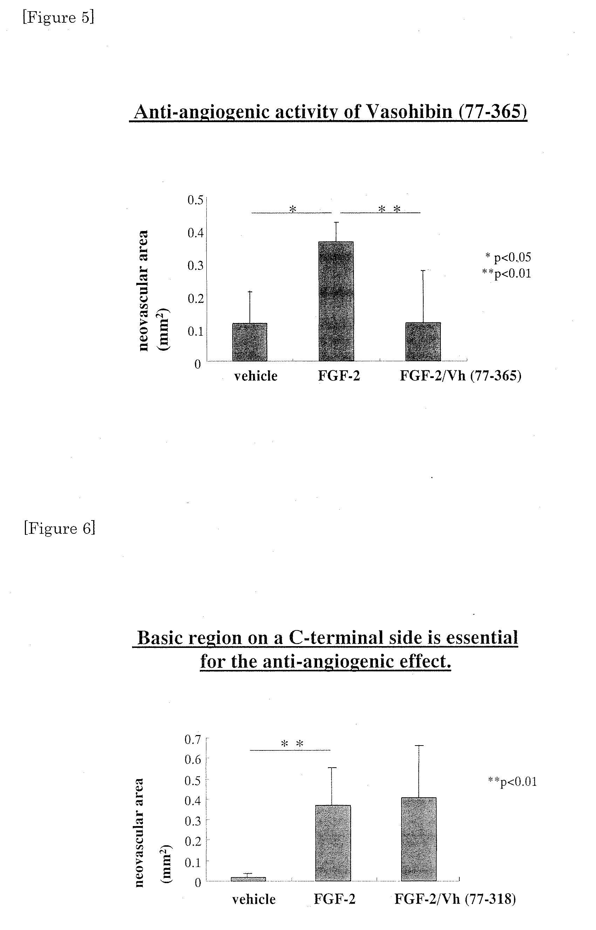

Whether the Vasohibin (77-365) protein or the Vasohibin (77-318) protein has the anti-angiogenic ability or not was studied by a mouse cornea micropocket assay according to the method described in J.Immunol. 170:5704-5711, (2003) and FASAB J.18:300-310, (2004). Into 0.3 µg of a Hydron reagent (IFN Sciences) was mixed 80 ng of the FGF-2 protein (fibroblast growth factor-2, BD Biosciences), 5 ng of the Vasohibin (77-365) protein or the Vasohibin (77-318) protein was further added, and this was transplanted into a cornea of a BALB/c male mouse. Seven days after, a new-born vessel extended in a cornea was photographed, and quantitative analysis of a angiogenesis was performed with an image analyzing software National Institutes of Health (NIH) image. As a result, angiogenesis induced by FGF-2 was significantly inhibited in the presence of the Vasohibin (77-365) protein (Fig.5), while anigogenesis was not inhibited in the presence of the Vasohibin (77-318) protein (Fig.6). Therefore, it was demonstrated that 319-position to 365-position amino acids are necessary in order that the wild-type Vasohibin protein or a polypeptide derived therefrom has the anti-angiogenic activity.

### Industrial Applicability

The polypeptide of the present invention, a derivative thereof, a pharmaceutically acceptable salt thereof or a solvate thereof can be used as an anti-angiogenic agent. A neutralizing antibody or an aptamer to the polypeptide or the like can be used as an angiogenesis inducing agent. In addition, an anti-anglogenic agent or an angiogenesis inducing agent can be screened using the polypeptide, the antibody or the like.

### Sequence listing free text

SEQ ID No.:1 indicates a nucleotide sequence of a gene encoding a vasohibin protein.

SEQ ID No.:2 indicates an amino acid sequence of a vasohibin protein.

## Claims

1. A polypeptide consisting of continuous not more than 364 amino acids of an amino acid sequence represented by Met at a 1-position to Val at a 365-position of SEQ ID No. 2, and comprising an amino acid sequence of Lys at a 319-position to Val at a 365-position, or a derivative thereof, or a pharmaceutically acceptable salt thereof or a solvate thereof.

2. The polypeptide according to claim 1, wherein the polypeptide comprises an amino acid sequence of Met at a 77-position to Val at a 365-position, or a derivative thereof, or a pharmaceutically acceptable salt thereof or a solvate thereof.

3. The polypeptide according to claim 1, wherein the polypeptide comprises an amino acid sequence of Arg at a 30-position to Val at a 365-position, or a derivative thereof, or a pharmaceutically acceptable salt thereof or a solvate thereof.

4. The polypeptide according to claim 1, wherein the polypeptide consists of an amino acid sequence starting at any amino acid of Arg at a 30-position to Arg at a 318-position and terminating at Val at a 365-position, or a derivative thereof, or a pharmaceutically acceptable salt thereof or a solvate thereof.

5. The polypeptide according to claim 1, wherein the polypeptide consists of an amino acid sequence starting at any amino acid of Met at a 77-position to Agr at a 318-position and terminating at Val at a 365-position, or a derivative thereof, or a pharmaceutically acceptable salt thereof or a solvate thereof.

6. The polypeptide according to claim 1, wherein the polypeptide consists of an amino acid sequence starting at any amino acid of Arg at a 30-position to Met at a 77-position and terminating at Val at a 365-position, or a derivative thereof, or a pharmaceutically acceptable salt thereof or a solvate thereof.

7. A polypeptide in which one or a few amino acid residues are deleted or substituted in the polypeptide as defined in any one of claims 1 to 6, wherein the polypeptide has an anti-angiogenic activity, or a derivative thereof, or a pharmaceutically acceptable salt thereof or a solvate thereof.

8. A method of screening a substance which binds to the polypeptide as defined in any one of claims 1 to 7, comprising the following;
(a) a step of contacting the polypeptide as defined in any one of claims 1 to 7 with a test substance, and
(b) a step of detecting binding of the polypeptide with the test substance.

9. A method of screening a binding activity regulating substance which regulates binding of the polypeptide as defined in any one of claims 1 to 7 with a substance which binds to the polypeptide, comprising the following;
(a) a step of contacting the polypeptide as defined in any one of claims 1 to 7 with a binding substance of the polypeptide in the presence or the absence of a test substance, and
(b) a step of comparing activity of binding of the polypeptide with the binding substance between in the presence and in the absence of the test substance.

10. A method of screening a substance which regulates expression of the polypeptide as defined in any one of claims 1 to 7, comprising the following;
(a) a step of contacting a cell which can express the polypeptide as defined in any one of claims 1 to 7 with a test substance, and
(b) a step of detecting a change in an expression amount of the polypeptide.

11. A pharmaceutical composition comprising the polypeptide as defined in any one of claims 1 to 7, or a derivative thereof, or a pharmaceutically acceptable salt thereof or a solvate thereof.

12. The pharmaceutical composition according to claim 11, which is a therapeutic agent for a vascular disease, an inflammatory disease, an entoptic neovascular disease, a reproductive system disease, a central nervous system disease or cancer.
